(19) Europäisches Patentamt
European Patent Office
Office européen des brevets

(11) **EP 2 858 568 B1**

(12) **EUROPÄISCHE PATENTSCHRIFT**

(45) Veröffentlichungstag und Bekanntmachung des Hinweises auf die Patenterteilung:
**13.07.2016 Patentblatt 2016/28**

(21) Anmeldenummer: **13731024.9**

(22) Anmeldetag: **24.05.2013**

(51) Int Cl.:
*B23K 26/38* (2014.01)   *A61B 5/145* (2006.01)
*A61B 5/1455* (2006.01)   *A61B 5/00* (2006.01)
*G01J 5/02* (2006.01)   *B29K 67/00* (2006.01)
*B29K 105/00* (2006.01)   *B29C 51/08* (2006.01)

(86) Internationale Anmeldenummer:
**PCT/AT2013/050112**

(87) Internationale Veröffentlichungsnummer:
**WO 2013/181681 (12.12.2013 Gazette 2013/50)**

(54) **ABDECKKAPPE UND MESSGERÄT**

COVERING CAP AND MEASURING DEVICE

CAPUCHON ET APPAREIL DE MESURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2012 AT 6622012**

(43) Veröffentlichungstag der Anmeldung:
**15.04.2015 Patentblatt 2015/16**

(60) Teilanmeldung:
**16161365.8**

(73) Patentinhaber: **VASEMA DIAGNOSTICS AG**
**8050 Zürich (CH)**

(72) Erfinder: **Peter Hagl**
**8143 Stallikon (CH)**

(74) Vertreter: **Wildhack & Jellinek**
**Patentanwälte**
**Landstraßer Hauptstraße 50**
**1030 Wien (AT)**

(56) Entgegenhaltungen:
**EP-A1- 1 262 753      EP-A2- 0 928 969**
**US-A- 4 207 160      US-A- 4 303 076**
**US-A1- 2001 034 479   US-A1- 2007 058 692**
**US-A1- 2010 329 951   US-A1- 2011 202 121**

**Beschreibung**

Die Erfindung betrifft eine Abdeckkappe, insbesondere zum Aufsetzen auf ein Hautanalysegerät gemäß dem Oberbegriff des Patentanspruches 1. Weiters betrifft die Erfindung ein Messgerät, insbesondere zur Hautanalyse gemäß Patentanspruch 15. Ferner betrifft die Erfindung ein Verfahren zur Herstellung einer Abdeckkappe gemäß dem Oberbegriff des Patentanspruches 23.

Hintergrund der Erfindung ist die Verwendung von Abdeckkappen, auch Hygienekappen genannt, zur Abdeckung von Sensoren eines Messgeräts. Hierbei sind Abdeckkappen unerlässlich, um steril oder unsteril, sauber Menschen, Tiere oder Materialien zu vermessen, ohne den mit der Abdeckkappe abgedeckten Sensor oder dass von der Abdeckkappe abgedeckte Messgerät zu verschmutzen. Solche Abdeckkappen werden im Stand der Technik, beispielsweise bei einem Infrarotthermometer verwendet, wobei der Infrarotsensor zum Schutz vor Verschmutzungen mit einer Abdeckkappe abgedeckt ist. Die Verwendung von Abdeckkappen dient dazu, Krankheiten nicht auf andere Menschen oder Körperteile zu übertragen. Dokumente US 2010/329951 und EP 1 262 753 offenbaren solche Abdeckkappen und Messgeräte.

Die aus dem Stand der Technik verfügbaren Abdeckkappen weisen jedoch in vielerlei Hinsicht Nachteile auf. Einerseits besteht das Problem, dass bekannte Abdeckkappen viel Raum benötigen und schlecht faltbar sind. Ferner besteht auch das Problem, mit Abdeckkappen hygienisch zu hantieren, sowie eine optimale Passform der Hygienekappe zu erzielen, insbesondere das Spannen der Abdeckkappe, um den sensitiven Teil eines Messgeräts ermöglichen. Weiters besteht das Problem, eine Abdeckkappe zu entsorgen, ohne diese zu berühren.

Aus dem Stand der Technik ist es bekannt, dass Hygienekappen auf einer möglichst gleichmäßigen und gleichdicken Material, vorzugsweise Polyethylen oder Polypropylen, hergestellt werden, wobei derjenige Oberflächenbereich der jeweiligen Abdeckkappe mittels eines Mikrotonmeters oder mittels Laser im Bereich der Stirnwand verdünnt wird, durch die der die jeweilige Temperatur charakterisierende Infrarotstrahl hindurch dringt.

Weiters kann die Verdünnung der Oberfläche im Bereich der Stirnwand auch mittels Extrudieren einer Schmelze mit anschließenden Einsetzen einer Folie verwirklicht werden. Die so erzielten Abdeckkappen sind sehr steif, was einerseits das Spannen der Abdeckkappen um das Messgerät erschwert und auch ein platzsparendes Recycling durch Zusammenfalten und Zusammendrücken auf einen flachen Gegenstand unmöglich macht.

Somit besteht die Aufgabe der Erfindung darin, eine Abdeckkappe zu schaffen, die das Messgerät vorteilhaft abdeckt und sich der Form des Messgeräts optimal anpasst und die einfach und mit geringem Volumen zu entsorgen ist. Die Erfindung löst diese Aufgabe bei einer Abdeckkappe der eingangs genannten Art mit dem kennzeichnenden Merkmal des Patentanspruchs 1.

Eine weitere Aufgabe der Erfindung liegt in der Bereitstellung eines vorteilhaften Verfahrens zur Herstellung einer Abdeckkappe. Die Erfindung löst diese Aufgabe beim Verfahren der eingangs genannten Art mit dem kennzeichnenden Merkmal des Patentanspruchs 23.

Weiters ist es Aufgabe der Erfindung ein Messgerät zu schaffen, das eine vorteilhafte und berührungsfreie Entsorgung mit einer erfindungsgemäßen Abdeckkappe ermöglicht. Die Erfindung löst diese Aufgabe bei einem Messgerät der eingangs genannten Art mit dem kennzeichnenden Merkmal des Patentanspruchs 15.

Erfindungsgemäß ist bei einer Abdeckkappe, insbesondere zum Aufsetzen auf ein Hautanalysegerät, umfassend eine von einer Umfangskante begrenzte Stirnwand sowie eine an der Umfangskante der Stirnwand anschließende Mantelwand, vorgesehen, dass die Stirnwand gasdurchlässig ausgebildet ist.

Eine besondere Weiterbildung der Erfindung sieht vor, dass die Mantelwand zumindest einen umlaufenden Abschnitt aufweist, dessen Dicke geringer ist als die Dicke der Mantelwand im Abschnitt des von der Stirnwand abgewandten Endes der Mantelwand. Durch diese Maßnahme ist im Bereich des umlaufenden Abschnitts eine gewisse Flexibilität gegeben, mit der eine optimale Anpassung der Abdeckkappe an das jeweilige Messgerät ermöglicht wird.

Ganz grundsätzlich ist es bei jeder Art von Abdeckkappe, insbesondere bei sämtlichen vorstehend genannten Weiterbildungen der Erfindung, von Vorteil, wenn dass die Stirnwand an zumindest einer Stelle eine Dicke im Bereich von weniger als 100 $\mu$m, vorzugsweise zwischen 0,1 $\mu$m und 20 $\mu$m, insbesondere zwischen 5 $\mu$m und 10 $\mu$m, aufweist. Hierdurch ist es möglich, eine Stirnwand zu erstellen, durch die Gase hindurch diffundieren können und eine Abdeckkappe auch zum Schutz des Sensors vor schädlichen Stoffen möglich ist, ohne die gesamte Funktion des Messgeräts zu beeinträchtigen, da durch die Ausbildung Stirnwand die zu messenden Stoffe durch diese diffundieren können.

Insbesondere kann vorgesehen sein, dass die Stirnwand insbesondere eine konstante Dicke aufweist. Hierdurch wird eine besonders genaue Messung ermöglicht.

Zur Erhöhung der Stabilität sowie der Standfestigkeit der Abdeckkappe kann vorgesehen sein, dass eine Grundwand, die die Mantelwand an dem von der Stirnwand abgewandten Ende, insbesondere einstückig, fortsetzt, und die vorzugsweise parallel oder in einem Winkel von höchstens 10° zur Stirnwand von der Mantelwand nach außen, insbesondere mit ansteigendem Normalabstand zur Stirnwand, verläuft, wobei die Grundwand vorzugsweise kreisringförmig ausgebildet ist und insbesondere einen Außenradius zwischen 2 mm und 50 mm und/oder einen Innenradius zwischen 1 mm und 45 mm aufweist und/oder eine Kreisringbreite von 0,5 mm bis 35 mm aufweist.

Eine besonders vorteilhafte Anpassung an Messgeräte mit einer Auswurfvorrichtung oder einem Auswurfelement sieht vor, dass die Mantelwand im Bereich der Umfangskante der Stirnwand in einem Winkel von zwischen 20° und 95°,insbesondere zwischen 60° und 80°, zur Stirnwand nach außen absteht, wobei sich de Mantelwand, insbesondere konisch, erweitert.

Eine vorteilhafter Aspekt zur Verbindung der Abdeckkappe mit einem Messgerät sieht vor,

- dass die Abdeckkappe in dem der Stirnwand abgewandten Ende der Mantelwand Rastausnehmungen und/oder Rastvorsprünge zur lösbaren Befestigung an einer Messvorrichtung vorgesehen sind, und/oder
- dass insbesondere die einzelnen Rastausnehmungen und/oder Rastvorsprünge jeweils denselben Normalabstand zur Stirnwand aufweisen und/oder in Umfangsrichtung der Mantelwand gleichmäßig verteilt sind, und/oder wobei die Gesamtzahl der Rastausnehmungen und/oder Rastvorsprünge zwei oder vier beträgt, und/oder
- dass insbesondere die Rastausnehmungen und/oder Rastvorsprünge in einem Winkel von 5° bis 40° und/oder höchstens 2 mm, insbesondee höchstens 0,3 mm von der Mantelwand abstehen oder in diese eindringen. Hierdurch wird es zusätzlich ermöglicht, die Abdeckkappe einfach und automatisiert vom Messgerät zu entfernen.

Ein weiterer bevorzugter Aspekt der Erfindung sieht vor, dass die Stirnwand strahlungs-, dampf-, feuchtigkeits-, partikel-, und/oder lichtdurchlässig ausgebildet ist und/oder eine Anzahl von, insbesondere dampfdurchlässigen oder gasdurchlässigen, Mikrolöchern aufweist. Hierdurch wird es möglich, die so ausgebildete Abdeckkappe für eine Vielzahl unterschiedlicher Messgeräte anwendbar zu machen.

Eine besonders anpassungsfähige, robuste und widerstandsfähige Weiterbildung der Erfindung sieht vor, dass die Abdeckkappe materialeinheitlich und/oder einstückig ausgebildet ist, und insbesondere aus thermoplastischem oder plastisch verformten oder verformbaren Kunststoff, vorzugsweise aus einem der folgenden Materialien oder einer Mischung hiervon, besteht:

- PETG (Polyethylenterephthalat Glycol),
- PP (Polypropylen),
- PE (Polyethylen),
- PC (Polycarbonat),
- PVC (Polyvinylchlorid),
- PS (Polystyrol),
- ABS (Acrylonitrile butadiene styrene),
- HDPE (High Density Polyethylene),
- LDPE (Low Density Polyethylene),
- PET (Polyethylene terephthalate),
- PMMA (Polymethyl methacrylate),
- ECOTERM S 900 T1,
- PETG / Copolyester 67639,

wobei das Material der Abdeckkappe (10), insbesondere zusätzlich, einen oder mehrere der folgenden Bestandteile enthält:

- Additive,
- Stabilisatoren,
- Farbmittel,
- Füllstoffe,
- Verstärkungsstoffe,

wobei insbesondere das Material der Abdeckkappe einen Farbstoff aufweist und die Stirnwand für bestimmte, insbesondere sichtbare, Wellenlängenanteile undurchsichtig oder absorbierend ausgebildet ist.

Eine besonders einfach herzustellende Abdeckkappe, die ein einfaches Zusammenfalten bzw. Zusammenklappen ermöglicht, sieht vor, dass die Dicke der Mantelwand von der Stirnwand zu dem von der Stirnwand fernen Ende der Mantelwand hin, insbesondere zumindest über einen Teilbereich der Mantelwand, vorzugsweise kontinuierlich, zunimmt. Eine im Bereich der Stirnwand besonders robuste Abdeckkappe, die ein einfaches Hantieren beim Aufsetzen auf das Messgerät ermöglicht, sieht vor, dass die Mantelwand im Nahebereich der Stirnwand einen, insbesondere an die Umfangskante anschließenden, ersten Verdickungsabschnitt aufweist, in dem die Dicke der Mantelwand die Dicke der Stirnwand übersteigt,

dass die Mantelwand einen an den ersten Verdickungsabschnitt anschließenden Zwischenabschnitt aufweist, wobei die Dicke der Mantelwand im Zwischenabschnitt geringer ist als die Dicke der Mantelwand im ersten Verdickungsabschnitt,

dass die Mantelwand einen an den Zwischenabschnitt anschließenden und bis zu dem der Stirnwand fernen Ende der Mantelwand reichenden zweiten Verdickungsabschnitt aufweist, und

dass die Dicke der Mantelwand im zweiten Verdickungsabschnitt größer ist als die Dicke der Mantelwand im Zwischenabschnitt.

Eine einfache Fertigung wird dadurch ermöglicht, dass die Dicke der Mantelwand, insbesondere in axialer oder radialer Richtung, einen über die Mantelwand kontinuierlichen, knick- und kantenfreien Verlauf aufweist, und/oder als Dicke die jeweils mittlere Dicke im jeweiligen Abschnitt der Mantelwand gilt, und/oder

dass die Mantelwand, insbesondere mit Ausnahme des Bereichs der Rastvorsprünge und/oder Rastausnehmungen rotationssymmetrisch ausgebildet ist.

Besonders vorteilhafte Ausprägungen und Weiterbildungen der Erfindung sehen vor, dass die Dicken des ersten Verdickungsabschnitts, des Zwischenabschnitts und des zweiten Verdickungsabschnitts zueinander im folgenden Verhältnis stehen:

$$2 < d_1 : d_2 < 5 \text{ und/oder } 2 < d_3 : d_2 < 5 \text{ und/oder } 0,8 < d_1 : d_3 < 1,25$$

und/oder dass die Dicken des ersten Verdickungsabschnitts, des Zwischenabschnitts und des zweiten Verdickungsabschnitts wie folgt festgelegt sind:

$$150 \ \mu m < d_1 < 250 \ \mu m \text{ und/oder } 50 \ \mu m < d_2 < 100 \ \mu m \text{ und/oder } 150 \ \mu m < d_3 < 250 \ \mu m.$$ Hierdurch wird eine besonders vorteilhafte Abdeckkappe ausgebildet, die in ihrem der Stirnfläche fernen Endbereich besonders stabil und robust ist, im Bereich der Stirnfläche eine erhöhte Stabilität und im Zwischenbereich zwischen den beiden Verdickungsabschnitten eine gute Elastizität und Komprimierbarkeit aufweist.

Eine besonders vorteilhafte Auswahl der einzelnen Höhen der Verdickungsabschnitte, sowie des Zwischenabschnitts sieht vor, dass die Höhe des ersten Verdickungsabschnitts, die Höhe des Zwischenabschnitts sowie die Höhe des zweiten Verdickungsabschnitts in folgendem Verhältnis stehen:

$$0,2 < h_1 : h_2 < 0,6 \text{ und/oder } 0,8 < h_3 : h_2 < 1,25 \text{ und/oder } 0,2 < h_1 : h_3 < 0,6.$$

Zur Abdeckung typischer Messgeräte ist es im vielen Fällen vorteilhaft, wenn die Mantelwand eine Höhe von 2mm bis 80mm aufweist und/oder

- dass das Verhältnis zwischen der Höhe und der maximalen Abmessung, insbesondere der Durchmesser oder die Diagonale, der Stirnwand (1) zwischen 0,01 und 55 liegt.

Um ein für den zu vermessenden Gegenstand oder für die zu vermessende Person besonders schonendes Messen zu ermöglichen, kann vorgesehen sein, dass die Stirnwand kreisförmig ausgebildet ist und insbesondere einen Durchmesser von 1 mm bis 50 mm aufweist, oder dass die Stirnwand quadratisch oder rechteckig

- ausgebildet ist und wobei die Seitenkanten der Stirnwand eine Länge von zwischen 2 mm und 40 mm aufweisen.

Weiters betrifft die Erfindung ein Messgerät insbesondere zur Hautanalyse, das mit einer Abdeckkappe mit Rastausnehmungen und/oder Rastvorsprüngen abdeckbar ist. Insbesondere ist das Messgerät mit einer erfindungsgemäßen Abdeckkappe abdeckbar und mit diesem verrastbar. Das Messgerät umfasst ein Gehäuse und einen darin angeordneten Sensor, wobei das Gehäuse einen konisch zulaufenden Gehäuseteil mit einer vorderen Stirnfläche aufweist, an dem eine Anzahl von Rastausnehmungen und/oder Rastvorsprüngen zum Verrasten mit den Rastausnehmunge und/oder Rastvorsprüngen der Abdeckkappe angeordnet ist,

insbesondere derart, dass im eingerasteten Zustand die Stirnwand der Abdeckkappe an der vorderen Stirnfläche anliegt. Mit diesem Messgerät ist es sehr einfach möglich, Abdeckkappen hygienisch und einfach durch Auswerfen bzw. Wegschießen der Abdeckkappe in einen dafür vorgesehenen Behälter zu entsorgen. Insbesondere ist es nicht erforderlich, die Abdeckkappe in dem Bereich zu berühren, mit dem sie auf dem zu vermessenden Gegenstand, oder auf dem zu vermessenden Lebewesen bei der Messung aufgesetzt war.

Gemäß einer baulich vorteilhaften Ausgestaltung zum Auswerfen der Abdeckkappe ist ein Auswurfelement vorgesehen, das in dem der vorderen Stirnwand entfernten Ende des konisch zulaufenden Gehäuseteils angeordnet ist und das von der vorderen Stirnwand weiter entfernt ist als die Rastausnehmungen und/oder Rastvorsprüngen,

- wobei das Auswurfelement zum Schieben einer eingerasteten Abdeckkappe durch Druckbeaufschlagung der Abdeckkappe in Richtung der vorderen Stirnwand ausgebildet ist und/oder

- wobei das Auswurfelement auf dem Gehäuse in Richtung der vorderen Stirnwand verschiebbar gelagert ist.

Um ein definiertes Auswerfen und Entsorgen der Abdeckkappe mit dem Messgerät zu erzielen, kann vorgesehen sein, dass an das Auswurfelement ein Hebelfortsatz anschließt oder von dem Auswurfelement ein Hebelfortsatz abgeht oder das Auswurfelement mit einem Hebelfortsatz verbunden ist,

- wobei der Hebelfortsatz in seinem Mittenbereich schwenkbar mit dem Gehäuse verbunden ist, sodass sich ein zweiarmiger Hebel ergibt, an dessen einen Ende das Auswurfelement angeordnet ist,
- wobei der am Gehäuse angelenkte Hebelfortsatz bei seinem Verschwenken, das Auswurfelement in Richtung der vorderen Stirnwand drückt.

Um ein vollständig berührungsloses Entfernen der Abdeckkappe vom Messgerät zu ermöglichen, kann vorgesehen sein, dass ein Auswurfelement, das in dem der vorderen Stirnwand entfernten Ende des konisch zulaufenden Gehäuseteils angeordnet ist und das von der vorderen Stirnwand weiter entfernt ist als die Rastausnehmungen und/oder Rastvorsprüngen, und

- dass das Auswurfelement zum Schieben einer eingerasteten Abdeckkappe durch Druckbeaufschlagung der Abdeckkappe in Richtung der vorderen Stirnwand ausgebildet ist und/oder
- dass das Auswurfelement auf dem Gehäuse in Richtung der vorderen Stirnwand (22) verschiebbar gelagert ist. Hierbei ist ein händisches Entfernen der Abdeckkappe vom Messgerät überhaupt nicht erforderlich und kann vielmehr durch Betätigung des Auswurfelements erfolgen.

Besonders einfach kann das Auswerfen erfolgen, wenn das Auswurfelement ringförmig, insbesondere kreisringzylinderförmig, ausgebildet ist und den konisch zulaufenden Gehäuseteil umgibt.

Eine besonders vorteilhafte Betätigung des Auswurfelements sieht vor, dass an das Auswurfelement ein Hebelfortsatz anschließt oder von dem Auswurfelement ein Hebelfortsatz abgeht oder das Auswurfelement mit einem Hebelfortsatz verbunden ist, wobei der Hebelfortsatz in seinem Mittenbereich schwenkbar mit dem Gehäuse verbunden ist, sodass sich ein zweiarmiger Hebel ergibt, an dessen einen Ende das Auswurfelement angeordnet ist, wobei insbesondere an dessen anderen Ende ein Betätigungselement vorgesehen ist,

wobei der am Gehäuse angelenkte Hebelfortsatz bei seinem Verschwenken, insbesondere durch Kraftbeaufschlagung des Betätigungselements, das Auswurfelement in Richtung der vorderen Stirnwand drückt.

Hierbei kann zur weiteren Verbesserung der Bedienbarkeit und Benutzbarkeit vorgesehen sein, dass das Auswurfelement und/oder der Hebelfortsatz durch ein Federelement mit einer Kraft oder Vorspannung beaufschlagt ist, die das Auswurfelement von der vorderen Stirnwand weg drückt oder weg zieht.

Ein besonders einfaches und wirkungsvolles Auswerfen der Abdeckkappe sieht vor, dass die einzelnen Rastausnehmungen und/oder Rastvorsprünge jeweils denselben Normalabstand zur Stirnfläche aufweisen und/oder in Umfangsrichtung der Mantelwand gleichmäßig verteilt sind, und/oder die Gesamtzahl der Rastausnehmungen und/oder Rastvorsprünge zwei oder vier beträgt, und/oder

dass die Rastausnehmungen und/oder Rastvorsprünge in einem Winkel von 10° bis 30° und/oder höchstens 1 mm, insbesondere höchstens 0,5 mm, von der Mantelwand abstehen und/oder

dass zwei Rastausnehmungen und/oder Rastvorsprünge vorgesehen sind, die denselben Abstand zu einem Punkt, in dem Bereich aufweisen, an dem der Hebelfortsatz an das Auswurfelement anschließt oder von diesem abgeht.

Eine Abdeckkappe mit einer besonders dünnen Stirnwand kann mit dem erfindungsgemäßen Verfahren hergestellt werden. Hierbei ist vorgesehen,

a) dass eine Kunststofffolie, insbesondere eine Folie bestehend aus PETG (Polyethylenterephthalat Glycol), als Ausgangsmaterial für einen Tiefziehprozess herangezogen wird, die vorzugsweise eine Dicke zwischen 0,04 mm und 0,5 mm aufweist,

b) dass ein Stempel mit einer Anzahl von separat beweglichen, aneinander anliegenden Teilstempeln auf die Kunststofffolie gepresst wird, wobei die einzelnen Teilstempel normal zur Ebene der Kunststofffolie bewegt werden und wobei die Kunststofffolie (31) durch den Druck der Teilstempel in eine Gegenform gedrückt wird und zu einer Kappenform verformt wird,

c) dass die einzelnen Teilstempel des Stempels während oder nach Schritt b) zueinander beabstandet werden, wobei jeder der Teilstempel von der Position des Stempels aus radial nach außen bewegt wird, wodurch in der Kunststofffolie eine Stirnwand, insbesondere mit einer Dicke im Bereich von weniger als 100 μm, vorzugsweise zwischen 3 μm und 20 μm, insbesondere zwischen 5 μm und 10 μm, ausgebildet wird.

Die Erfindung verwendet eine Abwandlung eines Tiefziehverfahrens, um besonders dünne Wandstärken der Stirnwand zu erreichen. Insbesondere die rotatorische Komponente der Ausformung der Abdeckkappen ermöglicht die Ausbildung sehr dünner Wandstärken.

Zur Ausbildung von Abdeckkappen mit unterschiedlichen Stärken der Mantelwand kann vorgesehen sein, dass anschließend an Schritt c) die Teilstempel aus der ausgebildeten Kappenform entfernt und ein weiterer Stempel mit einer ebenen Stirnfläche und einem sich erweiternden Stempelkörper, in das durch die Teilstempel ausgeformten Volumen der Kunststofffolie eingebracht wird, wobei die Kunststofffolie zwischen dem weiteren Stempel und einer zweiten Gegenform in eine Kappenform gebracht wird,

wobei vorzugsweise die Kunststofffolie, der weitere Stempel oder die zweite Gegenform nach dem Erhitzen der Kunststofffolie auf eine Temperatur zwischen 50°C und 90°C vorgeschoben wird.

Um die Wandstärken unterschiedlich ausbilden zu können, kann vorgesehen sein, dass zur Einstellung der jeweiligen Dicke der von der Stirnwand abgehenden Mantelwand die Vorschubgeschwindigkeit und/oder Temperatur der Teilstempel, des weiteren Stempels und/oder die Temperatur der Kunststofffolie und/oder die Temperatur der ersten oder zweiten Gegenform eingestellt wird,

wobei zur Verringerung der Wandstärke im Randbereich der durch den weiteren Stempel gebildeten Ausnehmung eine Erhöhung der genannten Temperaturen sowie eine Erhöhung der Vorschubgeschwindigkeit vorgenommen wird und wobei zur Erhöhung der Wandstärke im Randbereich der durch den weiteren Stempel gebildeten Ausnehmung eine Verringerung der genannten Temperaturen sowie eine Verringerung der Vorschubgeschwindigkeit vorgenommen wird.

Eine besonders vorteilhafte Abdeckkappe, die nach ihrer Verwendung gut zusammenfaltbar ist und vorteilhaft von einem Messgerät entfernt werden kann, kann hergestellt werden, indem die Temperatur des weiteren Stempels und/oder der Kunststofffolie sowie die Vorschubgeschwindigkeit des weiteren Stempels derart eingestellt werden,

dass während eines ersten Vorschubzeitraums des weiteren Stempels ein Abschnitt der Mantelwand mit einer vorgegebenen Dicke, insbesondere von 50 $\mu$m bis 100 $\mu$m, ausgebildet wird, und

dass während eines dem ersten Vorschubzeitraums nachfolgenden zweiten Vorschubzeitraums des weiteren Stempels ein Abschnitt der Mantelwand mit einer vorgegebenen Dicke, insbesondere von 150 $\mu$m und 250 $\mu$m, ausgebildet wird, die dicker ist als die Dicke der im ersten Vorschubzeitraum erstellten Mantelwand.

Hierbei kann vorteilhaft vorgesehen sein, dass die Wanddicke während des ersten Vorschubzeitraums des weiteren Stempels auf einen Wert eingestellt wird, der dem Doppelten bis Fünffachen der Wanddicke desjenigen Mantelbereichs entspricht, der bei der Ausformung der Stirnwand durch das Einbringen der Teilstempel ausgeformt wurde.

Zur Erstellung von Abdeckkappen mit kreisförmigen Stirnwänden kann vorgesehen sein, das dass die Teilstempel während ihrer radial von ihrem Zentrum weg gerichteten Bewegung in Schritt c) entlang einer um eine zentrale Achse spiralförmig nach außen verlaufenden und sich aufweitenden Kurve rotierend bewegt werden.

Eine besonders anpassungsfähige, robuste und widerstandsfähige Weiterbildung der Erfindung sieht vor, dass die Kunststofffolie aus thermoplastischem oder plastisch verformten oder verformbaren Kunststoff, vorzugsweise aus einem der folgenden Materialien oder einer Mischung hiervon, besteht:

- PETG (Polyethylenterephthalat Glycol),
- PP (Polypropylen),
- PE (Polyethylen),
- PC (Polycarbonat),
- PVC (Polyvinylchlorid),
- PS (Polystyrol),
- ABS (Acrylonitrile butadiene styrene),
- HDPE (High Density Polyethylene),
- LDPE (Low Density Polyethylene),
- PET (Polyethylene terephthalate),
- PMMA (Polymethyl methacrylate),
- ECOTERM S 900 T1,
- PETG / Copolyester 67639,

wobei das Material der Abdeckkappe, insbesondere zusätzlich, einen oder mehrere der folgenden Bestandteile enthält:

- Additive,
- Stabilisatoren,
- Farbmittel,
- Füllstoffe,
- Verstärkungsstoffe,

wobei insbesondere das Material der Abdeckkappe einen Farbstoff aufweist und die Stirnwand für bestimmte Wellenlängenanteile undurchsichtig oder absorbierend ausgebildet wird.

Zur Ausbildung von Merkmalen, insbesondere von Rastausnehmungen, im Innenbereich der Mantelfläche sowie auf der Stirnfläche kann vorgesehen sein, dass nach der teilweisen oder vollständigen Ausformung der Mantelwand die Teilstempel oder der weitere Stempel zurückgefahren werden und das ausgeformte Volumen mit, insbesondere erhitzter Druckluft, beaufschlagt wird und dass der weitere Stempel anschließend gegebenenfalls in das ausgeformte Volumen zur Durchführung des nächsten Ausformungsschritt eingebracht wird,

wobei insbesondere durch Beaufschlagung mit Druckluft Rastausnehmungen im Innenmantel der Mantelwand ausgeformt werden.

Um die erstellten Abdeckkappen vom Körper der Kunststofffolie zu entfernen, kann vorgesehen sein, dass nach der Ausformung der Mantelwand die an die Mantelwand anschließende verbleibende Kunststofffolie von der Mantelwand entlang einer vorgegebenen Schnittlinie abgetrennt wird, wobei die Schnittlinie insbesondere so gewählt wird, dass ein Teilabschnitt der an die Mantelwand anschließenden verbleibenden Kunststofffolie innerhalb der Schnittlinie liegt und eine Grundwand ausbildet.

Um die Durchlässigkeit der Folie festzulegen, kann vorgesehen sein, dass die Kunststofffolie, insbesondere nach ihrer Ausformung, für einen vorgegebenen Zeitraum, insbesondere für zumindest 3 Sekunden, auf ein vorgegebene Temperatur zwischen 30°C bis 120°C, insbesondere auf eine Temperatur zwischen 50°C und 90°C, aufgewärmt wird, und/oder

dass die Teilstempel und/oder der weitere Stempel und/oder die verwendeten Gegenformen vor der Bearbeitung der Kunststofffolie auf eine Temperatur zwischen 30°C bis 120°C, insbesondere auf eine Temperatur zwischen 50°C und 90°C, erwärmt werden und während der Bearbeitung auf einer solchen Temperatur gehalten werden.

Hierbei kann insbesondere vorgesehen sein, dass der die Kunststofffolie bildende Kunststoff durch die Erwärmung umgeformt wird, wobei dieser insbesondere seine thermoplastischen Eigenschaften verliert und/oder wobei vorzugsweise durch die Wärmeeinwirkung die die Kunststofffolie bildenden Molekülketten aufgespalten werden.

Um eine verbesserte Diffusion von Stoffen durch die Stirnwand zu ermöglichen, kann vorgesehen sein, dass, insbesondere mittels eines Lasers oder einer erhitzten Mikronadel oder Nanonadel oder mittels Ätzen, Löcher, insbesondere Mikrolöcher oder Nanolöcher, in der Stirnwand ausgebildet werden.

Mehrere Ausführungsbeispiele der Erfindung werden anhand der folgenden Zeichnungsfiguren dargestellt.

**Fig. 1a** zeigt eine erste Ausführungsform einer erfindungsgemäßen Abdeckkappe im Querschnitt. **Fig. 1b** zeigt die in **Fig. 1a** dargestellte Abdeckkappe von der Seite. **Fig. 2** zeigt eine Abdeckkappe gemäß einer besonders einfachen Ausführungsform der Erfindung im Querschnitt.

**Fig. 3** zeigt ein erfindungsgemäßes Messgerät. **Fig. 4** zeigt ein erfindungsgemäßes Messgerät mit aufgesetzter Abdeckkappe. **Fig. 4a** zeigt ein den Auswurf der Abdeckkappe vom Messgerät.

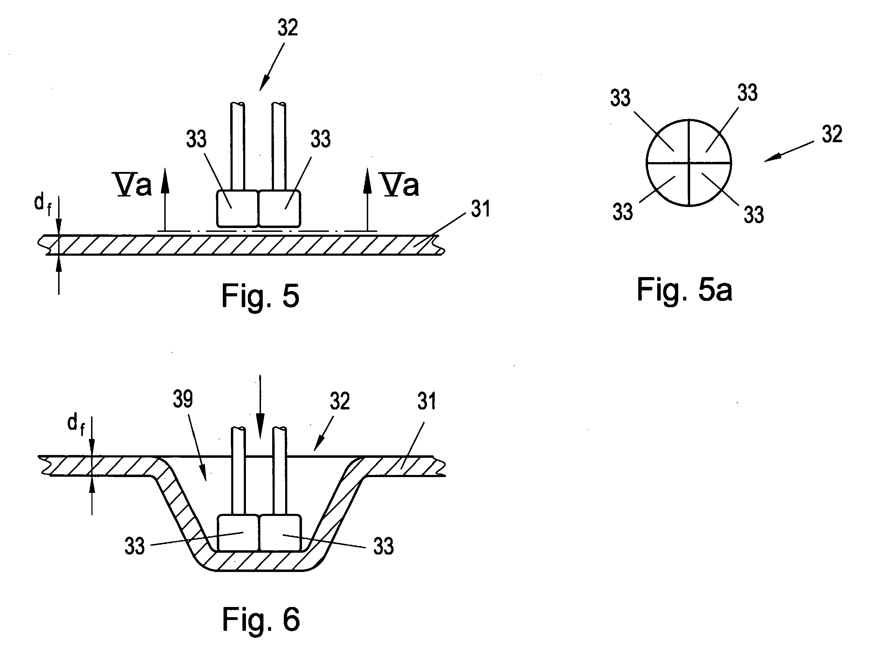

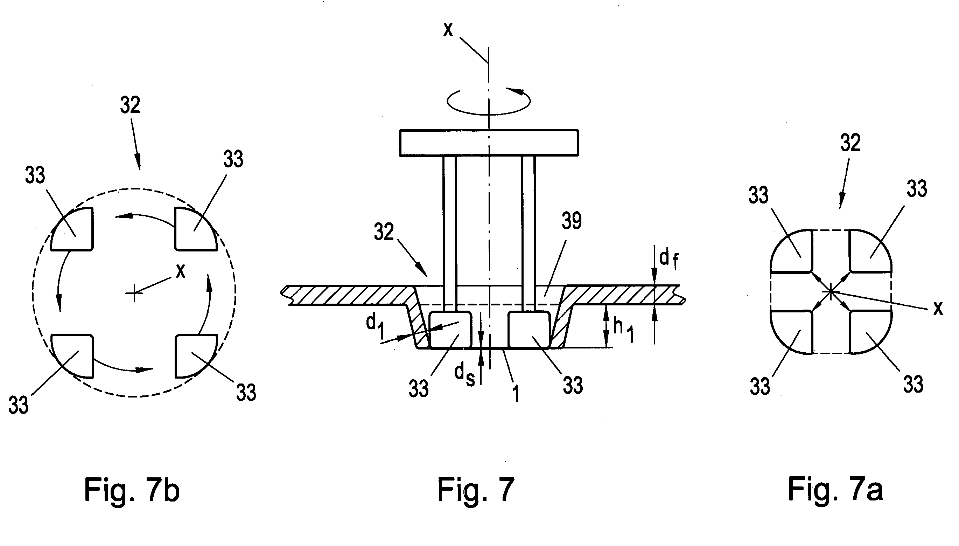

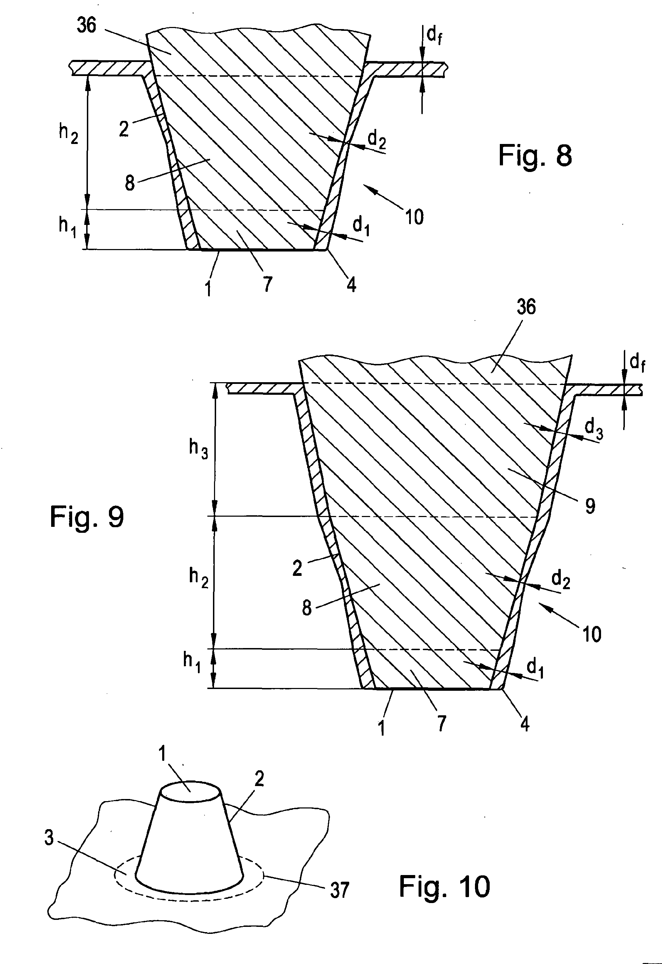

**Fig. 5** zeigt eine Kunststofffolie mit der die Abdeckkappe ausgebildet wird. **Fig. 5a** zeigt die Stirnfläche des Stempels und seiner Teilstempel. **Fig. 6** zeigt das Vordringen der Teilstempel in die Kunststofffolie. **Fig. 7** zeigt das Aufweiten der Teilstempel und die Ausbildung der Stirnfläche der Abdeckkappe. **Fig. 7a** zeigt die Bewegung der Teilstempel zur Ausformung einer rechteckigen Stirnfläche. **Fig. 7b** die Bewegung der Teilstempel zur Ausformung einer kreisförmigen Stirnfläche. **Fig. 8 und 9** zeigen die Ausbildung unterschiedlich dicker Mantelwandabschnitte der Abdeckkappe. **Fig. 10** zeigt die Fertigstellung der Abdeckkappe durch Abtrennen von der Kunststofffolie.

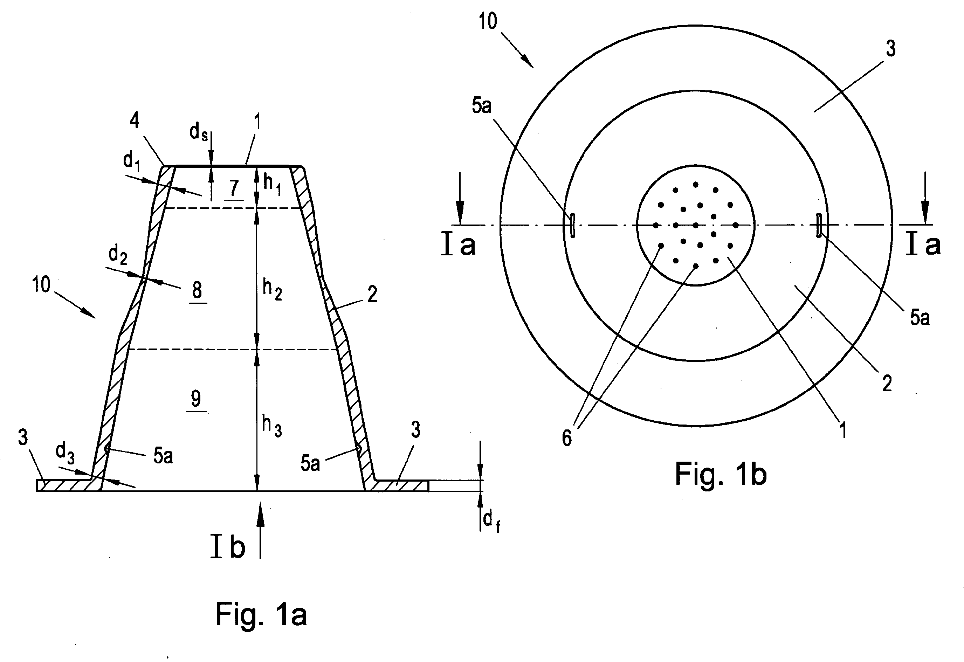

In **Fig. 1a** ist eine erste Ausführungsform einer erfindungsgemäßen Abdeckkappe 10 im Querschnitt (Schnittlinie **I a** in **Fig. 1b**) dargestellt. Dieselbe Abdeckkappe 10 ist in **Fig. 1b** von unten gemäß der in **Fig. 1a** angedeuteten Schnittlinie **I b** dargestellt. Diese Abdeckkappe 10 weist eine kreisrunde Stirnwand 1 auf, die von einer Umfangskante 4 begrenzt ist. An der Umfangskante 4 der Stirnwand 1 schließt eine Mantelwand 2 an, die an ihrem der Stirnwand 1 abgewandten Ende von einer Grundwand 3 fortgesetzt wird. Die Mantelwand 2 ist im Folgenden Ausführungsbeispiel sich annähernd konisch erweiternd ausgebildet und steht im Bereich der Umfangskante 4 in einen Winkel $\alpha$ von 75° von der Stirnwand 1 nach außen ab.

Die Stirnwand 1 weist eine sehr geringe Wanddicke $d_S$ von 10$\mu$m auf, vorteilhafterweise kann diese die Wanddicke $d_S$ in einem Bereich zwischen 0,05 $\mu$m und 20 $\mu$m insbesondere in einem Bereich von 3 $\mu$m bis 20 $\mu$m liegen. Die Stirnwand 1 weist im vorliegenden Fall einen Durchmesser von 5 mm auf. Grundsätzlich kann dieser Durchmesser in einem sehr weiten Bereich liegen, insbesondere vorteilhaft sind Durchmesser im Bereich zwischen 2 mm und 20 mm, wobei, je nach Anwendungsfalls, auch größere Durchmesser von bis zu 100 mm durchaus Anwendung finden können.

Eine alternative, nicht dargestellte Ausführungsform der Erfindung weist an Stelle eines kreisförmigen Aufbaus der Stirnwand 1 eine quadratische Stirnwand 1 auf, die im Wesentlichen eine gleichgroße Wandfläche aufweist, wie in dem in **Fig. 1** dargestellten Ausführungsbeispiel und weist eine Kantenlänge von etwa 10 mm auf. Alternativ kann die Stirnwand 1 auch rechteckig, quadratisch oder elliptisch ausgebildet sein, wobei die Seitenkanten der Stirnwand 1 etwa eine Länge zwischen 2 mm und 40 mm, insbesondere zwischen 5 mm und 8 mm, aufweisen können.

In dem in **Fig. 1a** dargestellten Ausführungsbeispiel ist der Mantel 2 der Abdeckkappe 10 dreiteilig ausgebildet und umfasst im Nahebereich der Stirnwand 1 einen an die Umfangskante 4 anschließenden, ersten Verdickungsabschnitt 7 mit einer Dicke $d_1$, im vorliegenden Ausführungsbeispiel wurde eine Dicke $d_1$ im Bereich zwischen 150 und 250 $\mu$m für den ersten Verdickungsabschnitt gewählt. Die Dicke $d_1$ der Mantelwand 2 im ersten Verdickungsabschnitt 7 übersteigt die Dicke $d_S$ der Stirnwand 1 und beträgt im vorliegenden Ausführungsbeispiel 5 $\mu$m bis 8 $\mu$m. Der erste Verdickungsabschnitt 7 ist umlaufend ausgebildet, wobei der erste Verdickungsabschnitt 7 auf dem von der Stirnwand 1 fernen Ende kreisförmig begrenzt ist, und wobei in diesem Begrenzungsbereich ein Zwischenabschnitt 8 an den ersten Verdickungsabschnitt 7 anschließt.

Im Zwischenabschnitt 8 weist die Mantelwand 2 eine Dicke $d_2$ auf, die geringer ist als die Dicke $d_1$ der Mantelwand 2 im ersten Verdickungsabschnitt 7. Im vorliegenden Ausführungsbeispiel wird als Dicke $d_2$ der Mantelwand 2 im Zwischenabschnitt 8 eine Dicke von 50 bis 150 $\mu$m gewählt. Der Zwischenabschnitt 8 ist auf seinem dem ersten Verdickungsabschnitt 7 fernen Ende kreisförmig begrenzt, wobei an diese kreisförmige Begrenzung ein zweiter Verdickungsabschnitt 9 anschließt, in dem die Dicke $d_3$ der Mantelwand 2 größer ist als die Dicke $d_2$ der Mantelwand 2 im Zwischenabschnitt 8. Im vorliegenden Ausführungsbeispiel wurde eine Dicke $d_3$ im Bereich zwischen 150 und 250 $\mu$m für den ersten Verdickungsabschnitt gewählt. Der zweite Verdickungsabschnitt 9 schließt im vorliegenden Fall an das der Stirnwand 1 ferne Ende der Mantelwand 2 an, insbesondere grenzt der zweite Verdickungsabschnitt 9 unmittelbar an die Grundwand 3 an.

Durch diese spezielle Ausbildung weist die Mantelwand 2 einen umlaufenden Abschnitt 8 in Form des Zwischenabschnittes 8 auf, in dem die Dicke $d_2$ geringer ist als die Dicke $d_3$ der Mantelwand 2 im Bereich des von der Stirnwand 1 abgewandten Endes der Mantelwand 2. Die Dicke der Mantelwand 2 nimmt in axialer bzw. radialer Richtung, ausgehend von der Dicke $d_1$ im ersten Verdickungsbereich 7 stetig bzw. kontinuierlich ab und erreicht im Zwischenabschnitt 8 ein Minimum, anschließend nimmt die Dicke der Mantelwand 2 im zweiten Verdickungsbereich 9 bis zu den der Stirnwand 1 fernen Ende der Mantelfläche zu.

Die Dicke der Mantelwand 2 weist einen über die Mantelwand 2 kontinuierlichen, knick- und kantenfreien Verlauf auf. Soweit für die folgenden Betrachtungen notwendig, wird jeweils als Dicke der Mantelwand 2 im jeweiligen Abschnitt 7, 8, 9 der Mantelwand 2 die mittlere Dicke in diesem Abschnitt verstanden.

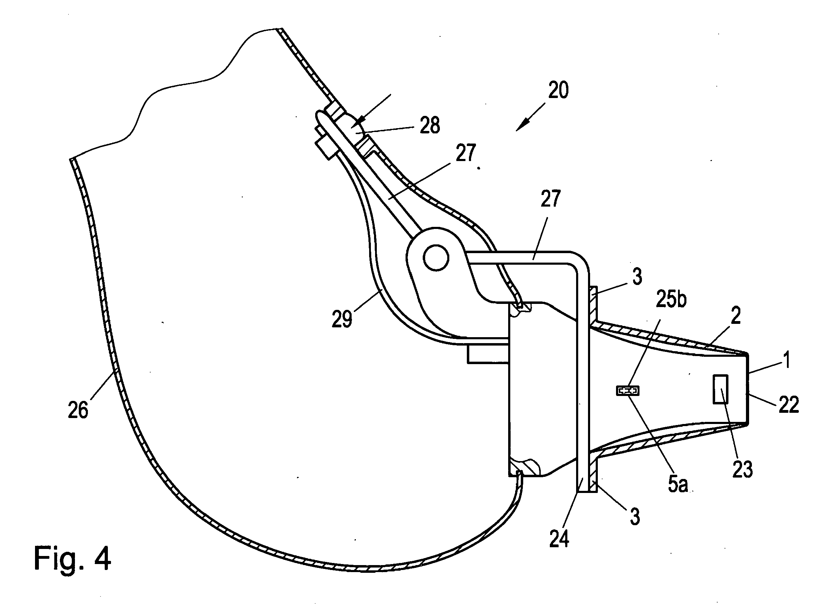

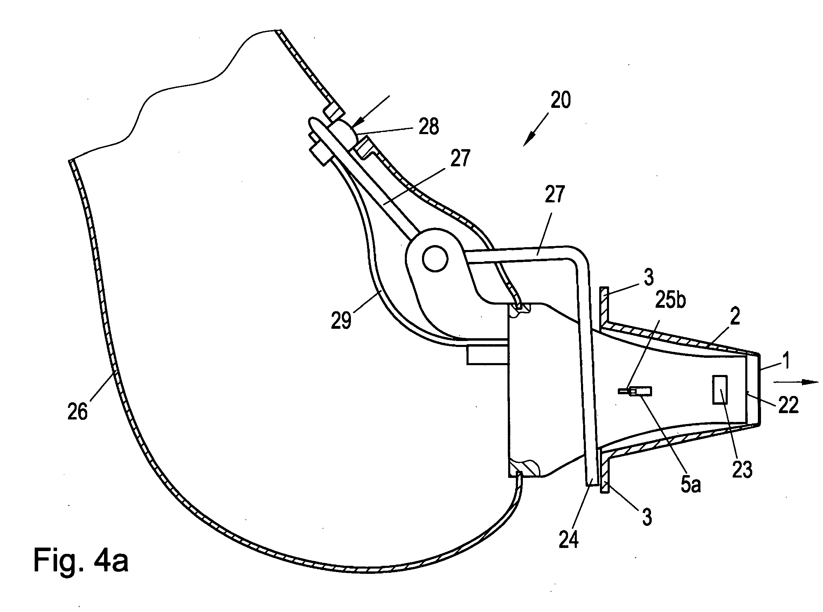

Um eine besonders vorteilhafte Anpassung der Stirnwand 1 an die Stirnfläche 21 **(Fig. 4)** eines Messgerätes 20 zu ermöglichen, ist die Dicke der Mantelwand 2 im Bereich des Zwischenabschnittes 8 dünner ausgebildet als in den übrigen beiden Verdickungsabschnitten 7, 9. Das Verhältnis der Dicke $d_2$ im Zwischenabschnitt 8 zur Dicke $d_1$ im ersten Verdickungsabschnitt 7 liegt etwa zwischen 2 und 5. Das Verhältnis der Dicke $d_2$ im Zwischenabschnitt 8 zur Dicke $d_3$ im zweiten Verdickungsabschnitt 9 liegt etwa zwischen 2 und 5. Die Dicke $d_1$ der Mantelfläche 2 im ersten Verdickungsabschnitt sowie die Dicke $d_3$ der Mantelfläche 2 im zweiten Verdickungsabschnitt 9 ist ungefähr gleichgroß. Das Verhältnis zwischen diesen beiden Dicken $d_1$, $d_3$ liegt etwa zwischen den Werten 0,8 und 1,25.

Im vorliegenden Ausführungsbeispiel weist die Mantelwand 2 als mittlere Dicke in den einzelnen Abschnitten 7, 8, 9 die folgenden Werte auf:

$$d_1 = 0{,}18\,\text{mm}$$

$$d_2 = 0{,}08\,\text{mm}$$

$$d_3 = 0{,}22\,\text{mm}$$

Die Höhe h der Mantelwand 2 beträgt im vorliegenden Ausführungsbeispiel 20 mm, sie kann jedoch, abhängig von der Form und vom Aufbau des abzudeckenden Messgerätes 20, unterschiedliche Werte aufweisen, die typischerweise zwischen 15 mm und 35 mm betragen. Das Verhältnis zwischen der Höhe h der Mantelwand 2 und der maximalen Abmessung der Stirnwand 1 liegt im Bereich zwischen 0,02 und 5.

Der erste Verdickungsabschnitt 7 ist im Bereich der Stirnwand 1 ausgebildet und weist im vorliegenden Ausführungsbeispiel eine Höhe von etwa 4 mm auf. Die übrigen beiden Abschnitte der Mantelwand 2, nämlich der Zwischenabschnitt 8 sowie der zweite Verdickungsabschnitt 9 weisen in etwa dieselbe Höhe auf, die im vorliegenden Fall jeweils ca. 10 mm beträgt. Typischerweise stehen die Höhe $h_1$ des ersten Verdickungsabschnittes 7, die Höhe $h_2$ des Zwischenabschnittes 8 sowie die Höhe $h_3$ des zweiten Verdickungsabschnittes 9 in folgendem Verhältnis zueinander:

$$0{,}2 < h_1 : h_2 < 0{,}6; \quad 0{,}8 < h_3 : h_2\ 1{,}25; \quad 0{,}2 < h_1 : h_3 < 0{,}6$$

Eine besondere weitere Ausgestaltung der Erfindung ermöglicht eine verbesserte Handhabung sowie eine geringere Riss- oder Bruchanfälligkeit der Abdeckkappe 20 beim Hantieren. Um eine bessere Handhabung zu gewährleisten, wird in der Abdeckkappe 10 im Bereich des zweiten Verdickungsabschnittes 9 bzw. in dessen unterem, der Stirnwand 1 fernem Endbereich ein Versteifungselement angeordnet. Ein solches Versteifungselement ist mit dem Innen- oder Außenmantel der Mantelfläche 2 verbunden und weist die Form einer Manschette auf, deren Oberflächenverlauf dem Oberflächenverlauf des Innen- oder Außenmantels entspricht. Diese Manschette besteht vorzugsweise aus versteiftem Kunststoff bzw. einem Kunststoff, der eine höhere innere Steifigkeit aufweist als der die Abdeckkappe 10 ausbildende Kunststoff.

Die Grundwand 3, die die Mantelwand 2 an dem von der Stirnwand 1 abgewanden Ende fortsetzt, verläuft im vorliegenden Ausführungsbeispiel parallel zur Stirnwand 1. Alternativ könnte die Grundwand 3 jedoch auch in einem Winkel von 10° zur Stirnwand 1 nach außen sowie von der Stirnwand 1 weg verlaufen. Vorzugsweise steht die Grundwand 3 von der Mantelwand 2 nach außen ab, wobei mit zunehmendem radialen Abstand der Normalabstand der Grundwand 3 von der Stirnwand ansteigt.

Die Grundwand 3 ist im vorliegenden Ausführungsbeispiel kreisringförmig und parallel zur Stirnwand 1 ausgebildet und weist einen Außendurchmesser von 21 mm und einen Innendurchmesser von 18 mm auf. Die Kreisringbreite beträgt im vorliegenden Fall 1,5 mm.

Weiters weist die Abdeckkappe 10 an der Innenseite der Mantelwand 2 Rastausnehmungen 5a auf, die eine lösbare Befestigung an einem Messgerät 20 (Fig. 4) ermöglichen. Im vorliegenden Ausführungsbeispiel sind zwei Rastausnehmungen 5b so angeordnet, dass sie einander an der Mantelwand der Abdeckkappe 10 gegenüber liegen. Alternativ können anstelle von Rastausnehmungen 5a auch nicht dargestellte Rastvorsprünge 5b vorgesehen sein. Grundsätzlich liegt der Zweck der Rastausnehmungen 5a und Rastvorsprünge 5b darin, eine Verrastung mit einem Messgerät 20 bzw. mit auf dem Messgerät 20 angeordneten Rastausnehmungen 25a und/oder Rastvorsprüngen 25b zu erzielen.

Die beiden Rastausnehmungen 5a sind im vorliegenden Ausführungsbeispiel als Ausnehmungen an der Innenseite der Mantelwand 2 im Bereich des zweiten Verdickungsabschnittes 9 ausgebildet und verlaufen etwa in einem Winkel $\beta_1$, $\beta_2$ von 20° gegenüber der Mantelwand 2. Die Rastausnehmungen 5a ragen etwa 60 $\mu$m in den zweiten Verdickungsbereich 9 hinein.

Alternativ besteht auch die Möglichkeit, anstelle von Rastausnehmungen 5b Rastvorsprünge 5a vorzusehen. Diese stehen von der Innenseite der Mantelwand 2 in einem Winkel $\beta_1$, $\beta_2$ von 10° bis 30° nach innen ab und stehen etwa 1,5 nm von der Mantelwand 2 nach innen ab. Die Rastvorsprünge 5a liegen einander auf derselben Höhe jeweils mit demselben Normalabstand zur Stirnwand 1 gegenüber und sind in Umfangsrichtung der Mantelwand 2 gleichmäßig verteilt, d.h. im vorliegenden Ausführungsbeispiel liegen sie einander um 180 gegenüber.

Alternativ besteht auch die Möglichkeit, dass die Rastausnehmungen 5a oder die Rastvorsprünge 5b in größerer Zahl vorhanden sind. Insbesondere erweist sich eine Anzahl von vier Rastvorsprüngen 5b oder Rastausnehmungen 5a als vorteilhaft; diese sind in Umfangsrichtung zueinander jeweils um 90° beabstandet.

In einer besonderen Ausführungsform der Erfindung kann vorgesehen sein, dass jeweils zwei der vier Rastelemente als Rastausnehmungen 5a, die übrigen als Rastvorsprünge 5b ausgebildet sind, wobei einander jeweils zwei Rastausnehmungen 5a und zwei Rastvorsprünge 5b um 180° umfangsseitig gegenüberliegen. Durch diese Maßnahme kann die Abdeckkappe 10 gegenüber einem Messgerät 20 **(Fig. 4)** vorteilhaft in ihrer Position gehalten werden, wobei die Mantelwand 2, insbesondere im Bereich des Zwischenabschnittes 8, einer elastischen Dehnung aufgrund der dünnen Dicke $d_2$ unterliegt und eine besonders gute Anpassung der Stirnwand 1 an eine abzudeckende Stirnfläche 21 **(Fig. 4)** des Messgerätes 20 ermöglicht wird. Das Ausmaß der Dehnung sowie die Dehnbarkeit der Mantelwand 2 sowie des Zwischenabschnitts 8 ist von dem verwendeten Kunststoff sowie von der Dicke $d_2$ der Mantelwand 2 im Bereich des Zwischenabschnitts 8 abhängig.

Weiters besteht auch die Möglichkeit, entweder am Messgerät 20 oder an der Abdeckkappe 10 umlaufende Rastelemente in Form von Rastvorsprüngen oder Rastrillen vorzusehen, wobei vorteilhafterweise ein umlaufendes Rastelement nur entweder auf der Abdeckkappe 10 oder auf dem Messgerät vorgesehen ist, um ein optimales Auswerfen der Abdeckkappe 10 vom Messgerät zu gewährleisten.

Im vorliegenden Ausführungsbeispiel ist die gesamte Abdeckkappe 10 materialeinheitlich ausgebildet und besteht aus Kunststoff. Im vorliegenden Fall wurde PETG verwendet, das mit einer geringen Menge an Farbstoffen vermischt ist. Die Verwendung von Farbstoffen für die Abdeckkappen 10 ermöglicht die Unterscheidung verschiedener Abdeckkappen mit unterschiedlichen Materialeigenschaften, insbesondere mit unterschiedlicher Dicke $d_S$ der Stirnwand 1. Es ist jedoch auch möglich, durch Beigabe von Farbstoffen die Stirnwand für bestimmte Wellenlängenanteile undurchsichtig oder absorbierend zu machen.

Anstelle von PETG (Polyethylenterephthalat Glycol) können auch aus dem Stand der Technik bekannte Kunststoffe, wie etwa Polyethylen oder Polypropylen oder Mischungen hieraus, verwendet werden.

In alternativen Ausführungsbeispielen der Erfindung die Abdeckkappe aus einer der folgenden Substanzen oder aus einer Mischung der folgenden Substanzen bestehen: PETG (Polyethylenterephthalat Glycol), PP (Polypropylen), PE (Polyethylen), PC (Polycarbonat), PVC (Polyvinylchlorid), PS (Polystyrol), ABS (Acrylonitrile butadiene styrene),

HDPE (High Density Polyethylene), LDPE (Low Density Polyethylene), PET (Polyethylene terephthalate), PMMA (Polymethyl methacrylate), ECOTERM S 900 T1. Hierbei können dem Material der Abdeckkappe 10 insbesondere zusätzlich einer oder mehrere der folgenden Bestandteile zugesetzt sein: Additive, Stabilisatoren, Farbmittel, Füllstoffe, Verstärkungsstoffe, PETG / Copolyester 6763.

Durch die konkrete Auswahl der Kettenlänge der einzelnen Molekülketten des Polyethylens, Polypropylens oder PETG kann festgelegt werden, für welche chemischen Verbindungen die Stirnwand 1 durchlässig ist bzw. für welche chemischen Verbindungen die Stirnwand undurchlässig ist. So besteht etwa die Möglichkeit, bestimmte Proben wie Blut, Urin, Schweiß oder Kot sowie lebende Proben, beispielsweise Haut, zu vermessen, wobei nur bestimmte, für die Messung gewünschte Inhaltsstoffe durch die Stirnwand 1 diffundieren können, etwa Natrium, Kalium, Chlor, Magnesium Vitamine, Hormone, Glycose, Alkohol, Spurenelemente und Wasserdampf, andere Bestandteile der Probe jedoch nicht oder nur nach sehr langer Zeit im Bereich Stunden durch die Stirnwand 1 diffundieren können, wie etwa Wasser, Blut, Urin oder Kot. Durch die Wahl kürzerer Ketten entstehen Löcher im jeweiligen Kunststoff oder in der jeweiligen Kunststoffstruktur, sodass größere Moleküle- durch die Stirnwand 1 diffundieren können. Je länger die Kettenmoleküle gewählt werden, desto engmaschiger verläuft die Kunststoffstruktur und desto kleiner müssen Moleküle sein, um die Stirnwand 1 durchdringen zu können. Weiters entscheidet auch die Dicke $d_S$ der Stirnwand 1 über die Durchlässigkeit, da Moleküle leichter durch eine dünnere Stirnwand 1 diffundieren können, als durch eine dickere Stirnwand 1. Sofern aufgrund von Vorgaben betreffend die Stabilität der Stirnwand 1 eine bestimmte Dicke erforderlich ist, können alternativ Mikrolöcher oder Nanolöcher 6 mit einem Durchmesser von 0,1 nm bis 5 $\mu$m, allenfalls bis 400 $\mu$m, in die Stirnwand 1, beispielsweise mit einer Mikronadel oder einem Laserstrahl, erstellt werden.

Alternativ können auch mit chemischen Prozessen wie etwa durch Ätzen Löcher 6 in der Stirnfläche 1 erzielt werden. Um eine weitere Beschädigung der Stirnfläche 1 zu vermeiden, wird diese im Anschluss an die Erstellung der Löcher vom jeweiligen Ätzmittel gereinigt, insbesondere abgespült.

Da die Molekülgrösse von Wasser ist 0,3 nm und die Molekülgrösse von Wasserdampf ist 0,1 nm beträgt, kann durch Vorsehen von Löchern mit einem Durchmesser von weniger als 0,3 nm Wasser nicht durch die Stirnfläche dringen. Ab einem Durchmesser der Löcher bzw. Poren von 1 nm gelangt Wasser langsam durch die Stirnfläche 1 ins Innere der Hygienekappe. Bei einer Messdauer von 3 Sekunden können die Löcher bzw. Poren auch mit 3 - 4 nm gewählt werden, da das Wasser weniger rasch durch die Stirnfläche diffundiert als der Dampf. Wird eine Abdeckkappe 10 mit Löchern mit einer Dicke von etwa 3-4 nm mit der Stirnfläche 1 voran in Wasser getaucht, benötigt dieses je nach Dicke der Stirnfläche etwa 1 Minute bis es die Stirnfläche 1 durchdrungen hat.

Aufgrund der Materialeinheitlichkeit weisen die einzelnen Teile der Abdeckkappe 10 im Wesentlichen dieselben Eigenschaften auf. Einzig durch die jeweilige Dicke $d_S$ der Stirnwand 1, der Mantelwand 2 sowie der Grundwand 3 können spezielle Durchlässigkeiten für bestimmte Stoffe oder Strahlungen erzielt werden. So kann die Stirnwand 1 im Zuge des Herstellungsverfahrens besonders dünn ausgebildet werden, damit diese die Diffusion oder Strömung bestimmter Materialien, wie Gase, Flüssigkeiten, Atome, Moleküle, Verbindungen, zulässt.

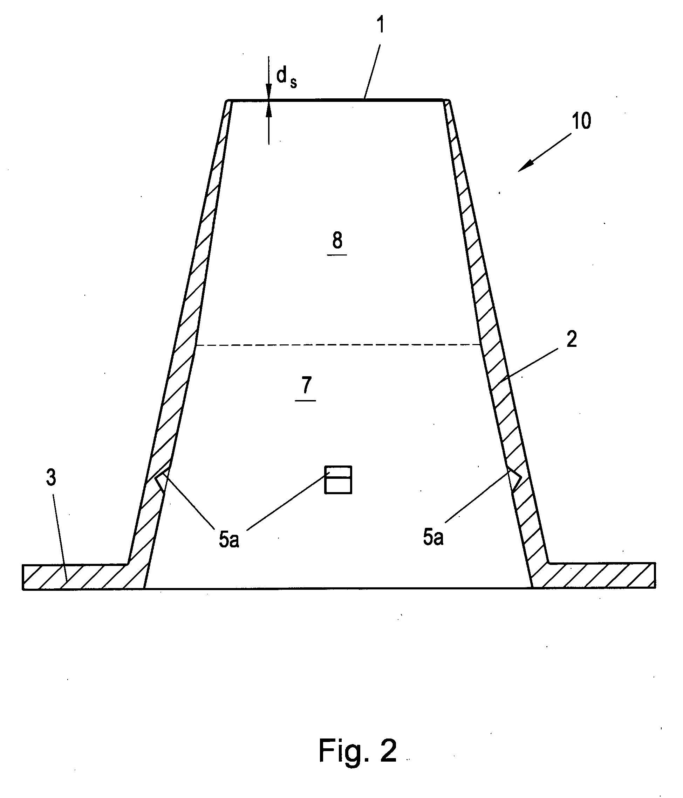

Das in **Fig. 2** dargestellte Ausführungsbeispiel entspricht im Wesentlichen dem in **Fig. 1a** und **Fig. 1b** dargestellten Ausführungsbeispiel, sodass lediglich auf die Unterschiede zwischen den beiden dargestellten Ausführungsbeispielen im Detail eingegangen wird. Die Höhe $h_1$ des an die Stirnwand 1 anschließenden Abschnittes 8 entspricht etwa der Höhe $h_2$ des der Stirnwand 1 fernen Verdickungsabschnittes 7.

Die Mantelwand 2 ist im vorliegenden Ausführungsbeispiel in zwei Abschnitte 7, 8 unterteilt, wobei beide Abschnitte 7, 8 umlaufend ausgebildet sind und die Abschnitte 7, 8 aneinander angrenzen und zwischen diesen beiden Abschnitten 7, 8 eine kreisförmige Begrenzungslinie verläuft, deren Punkte jeweils zur Stirnwand 1 denselben Normalabstand aufweisen. Die Dicke der Mantelwand 2 nimmt von der Stirnwand 1 aus, in axialer oder radialer Richtung gesehen, zu dem von der Stirnwand 1 fernen Ende der Mantelwand 2 hin kontinuierlich zu.

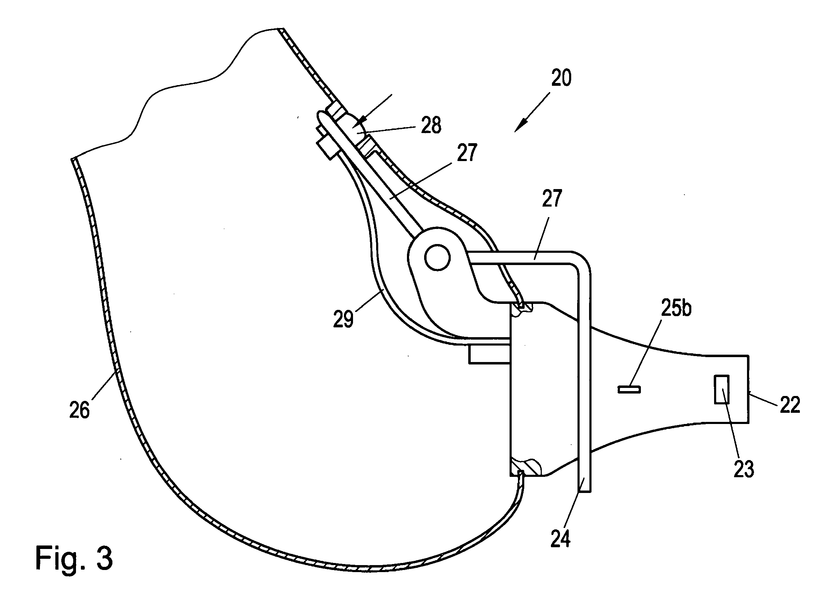

In **Fig. 3** ist eine Ausführungsform eines erfindungsgemäßen Messgerätes 20 näher dargestellt. Dieses Messgerät 20 weist ein Gehäuse 26 auf, das einen konisch zulaufenden Gehäuseteil 21 umfasst, der in weiterer Folge von einer vorstehend dargestellten Abdeckkappe 10 **(Fig. 4)** abgedeckt werden soll. Am Ende des konisch zulaufenden Gehäuseteils 21 ist eine Stirnfläche 22 angeordnet, an bzw. hinter der sich ein Sensor 23 befindet. Im vorliegenden Ausführungsbeispiel weist der konisch zulaufende Gehäuseteil 21 im Bereich der Stirnfläche 22 eine Öffnung auf, die zum Sensor 23 führt, sodass die zu messenden Gase und/oder Substanzen unmittelbar zum Sensor 23 strömen kann. Mit einer auf den konischen Gehäuseteil 21 aufgesetzten Abdeckkappe 10 **(Fig. 4),** kann gewährleistet werden, dass der Sensor 23 von Verschmutzungen frei gehalten wird.

Auf dem konisch zulaufenden Gehäuse 21 befindet sich eine Anzahl von Rastvorsprüngen 25b, alternativ oder zusätzlich kann auch eine Anzahl von Rastvorsprüngen 25a an der Mantelfläche des konisch zulaufenden Gehäuseteils 21 angeordnet sein. Wie in **Fig. 4** dargestellt, kann auf den konisch zulaufenden Gehäuseteil 21 die Abdeckkappe 10 aufgesetzt werden, sodass die vom konisch zulaufenden Gehäuseteil 21 abstehenden Rastvorsprünge 25b in die Rastausnehmungen 5a der Abdeckkappe 10 eingreifen. Um diesen abgedeckten Zustand des Messgeräts 20 zu erreichen, wird die Abdeckkappe 10, vorteilhafterweise mit zwei Fingern im unteren Verdickungsbereich 9 gegriffen und auf den konisch zulaufenden Gehäuseteil 21 aufgesetzt bzw. übergestülpt, sodass die Rastvorsprünge 25b des konisch zulaufenden Gehäuseteils 21 des Messgeräts in die Rastausnehmungen der Mantelwand 2 der Abdeckkappe 10 eingreifen. Die Abdeckkappe 10 ist dabei derart an den konisch zulaufenden Gehäuseteil 21 angepasst, dass sich die Rastvorsprünge bzw. Ausnehmungen 5a, 5b, 25a, 25b an aneinander angepassten Stellen befinden, sodass die Abdeckkappe 10 bzw. der konisch zulaufende Gehäuseteil 21 im verrasteten Zustand derart angeordnet sind, dass die Stirnfläche 1 über die Stirnwand 22 gestülpt ist und die an der Stirnfläche 22 des konisch zulaufenden Gehäuseteils 21 befindliche Ausnehmung vor dem Sensor 23 vollständig durch die Stirnwand 1 verdeckt oder abgedeckt wird. Dies wird insbesondere dadurch erreicht, dass die Abdeckkappe 10 in ihrem jeweiligen Abschnitt 8 mit geringer Dicke $d_2$ einer besonders starken elastischen Verformung unterliegt und somit eine optimale Passform für das jeweilige Messgerät bzw. dessen konisch zulaufenden Gehäuseteil 21 ausbildet.

Die Lage und Position der Rastausnehmungen 25a und Rastvorsprünge 25b auf dem konisch zulaufenden Gehäuseteil 21 entspricht der Position der Rastausnehmungen 5a und/oder Rastvorsprünge 5b auf der Abdeckkappe 10a. Insbesondere sind die Rastausnehmungen 25a und die Rastvorsprünge 25b jeweils mit den selben Normalabstand zur Stirnfläche 22 auf dem konisch zulaufenden Gehäuseteil 21 angeordnet und in Umfangsrichtung vorteilhafterweise gleichmäßig verteilt. Die Gesamtzahl der Rastausnehmungen und/oder Rastvorsprünge 25a, 25b beträgt im vorliegenden Ausführungsbeispiel zwei, Altenativ können jedoch auch drei oder vier oder auch mehr Rastausnehmungen und/oder Rastvorsprünge vorgesehen sein. Die Form der Rastausnehmungen oder Rastvorsprünge 25a, 25b ist jeweils an die Form der Abdeckkappen 10 bzw. der Rastausnehmungen 5a bzw. Rastvorsprünge 5b der Abdeckkappe 10 angepasst. Die Rastvorsprünge 25a und stehen in einem Winkel von 10° bis 30°, höchstens 1mm, insbesondere höchstens 0,5mn von der Außenwand des konisch zulaufenden Gehäuseteils 21 ab.

Das Messgerät 20 verfügt darüber hinaus über ein Auswurfelement 24, das in dem der vorderen Stirnwand 22 entfernten Ende des konisch zulaufenden Gehäuseteils angeordnet ist. Dieses Auswurfelement 24 ist von der vorderen Stirnwand 22 weiter entfernt als die Rastvorsprünge 25 des konisch zulaufenden Gehäuseteils. Das Auswurfelement 24 ist im vorliegenden Ausführungsbeispiel ringförmig, nämlich kreisringzylinderförmig, ausgebildet und umgibt den konisch zulaufenden Gehäuseteil 21. Wie aus Fig. 4 ersichtlich, liegt die Grundwand 3 der Abdeckkappe 10 auf dem Auswurfelement 24 flächig auf. Eine Druckbeaufschlagung des Auswurfelements 24 zur Stirnwand 22 hin, bewirkt, dass die Abdeckkappe 10 aus ihrer Verrastung geschoben wird, wobei die Abdeckkappe im Bereich ihrer Rastausnehmung 5a während des Verschiebens aus der Verrastung einer elastischen Verformung unterliegt. Aufgrund des abgewinkelten Aufbaus der Rastausnehmung 5a sowie des Rastvorsprungs 25b wird beim Ausrasten die bei der elastischen Verformung der Abdeckkappe 10 aufgewendete Energie in Bewegungsenergie der Abdeckkappe 10 umgewandelt, welche die Abdeckkappe nach einer Richtung vom Auswurfelement 24 zur Stirnwand 21 hin beschleunigt.

Im vorliegenden Ausführungsbeispiel ist das Auswurfelement 24 einstückig mit einem Hebelfortsatz 27 ausgebildet. Der Hebelfortsatz 27 schließt an das Auswurfelement 24 an oder geht von diesem ab und ermöglicht eine Verschiebung oder Verschwenkung des Auswurfelements 24 zur Stirnwand 22 hin. Der Hebelfortsatz 27 ist in seinem Mittelbereich schwenkbar mit dem Gehäuse 26 verbunden, wobei der Hebelfortsatz 27 als zweiarmiger Hebel fungiert. An einem Ende dieses zweiarmigen Hebels ist das Auswurfelement 24 angeordnet, am anderen Ende des zweiarmigen Hebels ist ein Betätigungselement 28 in Form eines Druckknopfs angeordnet. Wird der Druckknopf gedrückt, so wird das dem Auswurfelement 24 ferne Ende des Hebelfortsatzes 27 in der Darstellung der **Fig. 4a** entgegen dem Uhrzeigersinn verschwenkt und das Auswurfelement 24 wird aufgrund der Schwenkbewegung zur vorderen Stirnwand 22 hin gedrückt. Aufgrund dieser Druckbeaufschlagung der Abdeckkappe 10 rasten die eingerasteten Rastausnehmungen bzw. Rastvorsprünge 25a, 5b aus ihrer eingerasteten Stellung aus und die Abdeckkappe 10 wird in Richtung der vorderen Stirnwand 22 weggeschleudert.

Durch die konkrete Anlenkung des Auswurfelements 24 aufgrund der Schwenkbewegung kann die Flugbahn der Abdeckkappe 10 besonders präzise eingestellt werden. Durch die besondere schwenkbare Ausbildung des Auswurfelements 24 werden die in diesem Ausführungsbeispiel einander gegenüberliegenden aneinander angepassten Kombinationen von Rastausnehmungen und Rastvorsprüngen zu unterschiedlichen Zeitpunkten gelöst. Hierdurch wird die Abdeckkappe 10 nicht gerade ausgeworfen sondern erfährt eine hiervon abweichende Flugbahn, die bei normaler Handhaltung des Geräts nach unten gerichtet ist und die Entsorgung gebrauchter Abdeckkappen erleichtert.

Eine weitere vorteilhafte Anordnung von Rastausnehmungen oder Rastvorsprüngen 25a, 5b kann, wie im Folgenden dargestellt, verwirklicht werden, indem ausschließlich zwei Rastausnehmungen oder Rastvorsprünge 25a, 5b vorgesehen sind, die denselben Abstand zu demjenigen Punkt aufweisen, an dem der Hebelfortsatz 27 an das Auswurfelements 24 anschließt oder von diesem abgeht. Hierdurch wird gewährleistet, dass die Verrastung der Abdeckkappe 10 mit dem Messgerät 20 für sämtliche miteinander verrasteten Rastausnehmungen und Rastvorsprünge 5b, 25a gleichzeitig ausrastet und keine Verlangsamung durch ein erneutes Einrasten eines anderen Rastvorsprungs in eine Rastausnehmung und erfolgt.

Somit kann eine möglichst große Abschussweite von Abdeckkappen mit dem jeweiligen. Messgerät 20 erzielt werden. Dies hat den Vorteil, dass eine Entsorgung der Abdeckkappe über eine größere Distanz hinweg möglich ist und sich aufgrund der größeren Abschusskraft ein verbessertes Bediengefühl beim Entsorgen der Abdeckkappe einstellt und ganz allgemein die Motivation zur Benutzung sowie die Nutzerzufriedenheit gesteigert wird.

Um ein vorzeitiges oder unbeabsichtigtes Abschießen bzw. Entfernen einer Abdeckkappe aus dem verrasteten Zustand vom Messgerät 20 zu verhindern, ist im vorliegenden Ausführungsbeispiel eine Feder 29 in Form einer Drahtfeder vorgesehen, die den Hebelfortsatz 27 mit einer Vorspannung beaufschlagt, die das Auswurfelement 24 von der vorderen Stirnwand 22 wegdrückt. Erst wenn der Druck auf das Betätigungselement 28 die durch die Feder 29 erzeugte Vorspannung übersteigt wird der Abschuss der Abdeckkappe 10 veranlasst. Jedenfalls wird hierdurch vermieden, dass es zu einem unbeabsichtigten Abwerfen oder Auswurf der Abdeckkappe kommt.

Alternativ könnte diese Feder 29 auch an dem Auswurfelement 24 zugewandten Teil des Hebelfortsatzes 27 angreifen oder auch unmittelbar am Auswurfelement 24 selbst angreifen und dieses von der vorderen Stirnwand 22 wegziehen.

Im Folgenden wird ein Ausführungsbeispiel eines erfindungsgemäßen Verfahrens zur Herstellung einer Abdeckkappe 10 aus einer Kunststofffolie 31 dargestellt, wobei konkret die Herstellung des in den **Fig. 1a** und **Fig. 1 b** dargestellten Ausführungsform einer erfindungsgemäßen Abdeckkappe erläutert wird.

**Fig. 5** zeigt eine Kunststofffolie 31 als Ausgangsmaterial, mit der die Abdeckkappe 10 ausgebildet werden soll. Die Kunststofffolie 31 weist im Ausgangszustand eine Dicke $d_1$ von 0,4 mm auf und besteht aus Polyethylenterephthalat Glycol (PETG).

Zur Ausformung einer Stirnfläche 1 mit einer Dicke $d_S$ von etwa 1 $\mu$m bis 10 $\mu$m wird ein Stempel 32 umfassend eine Anzahl von mehreren Teilstempeln 33 verwendet. Im vorliegenden Ausführungsbeispiel umfasst der Stempel 32 vier Teilstempel 33. Der Stempel 32 ist zylindrisch ausgebildet weist eine kreisförmige Stirnfläche mit einem Radius 1 mm auf. Jeder Teilstempel 33 weist eine kreissegmentförmige Stirnfläche in Form eines Viertelkreises mit einem Radius von 1 mm auf. Alle Teilstempel 33 sind separat zueinander verschiebbar und verschwenkbar angeordnet. In **Fig. 5a** ist eine Sicht auf die Teilstempel 33 von der Kunststofffolie 31 aus, wie durch die Schnittlinie **V a** angedeutet, dargestellt.

In einem ersten Ausformungsschritt liegen die Teilstempel 33 aneinander an, die viertelkreisförmigen Stirnflächen der Teilstempel 33 bilden gemeinsam eine kreisförmige Stirnfläche aus. Die Teilstempel 33 werden in dieser Stellung gemeinsam in den Bereich der Kunststofffolie 31 vorgeschoben. Wie in **Fig. 6** dargestellt, wird die Kunststofffolie 31 hierdurch verformt. Es wird ein Volumen 39 ausgebildet, das bis auf diejenige Seite, von der die Teilstempel 33 her in die Kunststofffolie 31 eingedrungen sind, von der Kunststofffolie 31 begrenzt ist. Die Außenform der aus der Kunststofffolie 31 ausgebildeten Form wird durch eine nicht dargestellte erste Gegenform festgelegt, die an der äußeren Mantelwand 2 sowie an der Stirnwand 1 der aus der Kunststofffolie 31 ausgebildeten Abdeckkappe 10 anliegt.

Im nächsten Schritt, dargestellt in **Fig. 7,** werden die Teilstempel 33 radial nach außen bewegt. Im vorliegenden Fall wird die Kunststofffolie 31 im Bereich der Teilstempel 33 gleichzeitig auf eine Temperatur von 40 °C erwärmt. Hierdurch wird die Kunststofffolie 31 im Zwischenbereich zwischen den Teilstempeln 33 stark gezerrt und verdünnt, sodass sich eine Stirnfläche 1 mit eine Dicke $d_S$ von etwa 4 $\mu$m ergibt.

Grundsätzlich ist vorgesehen, dass die Teilstempel 33 zunächst eine axiale Bewegung normal zur Ebene der Kunststofffolie 31 vollführen **(Fig. 6)** und erst wenn die Teilstempel 33 in dieser Richtung eine Endposition eingenommen haben, die Teilstempel 33 in radialer Richtung **(Fig. 7)** verschoben werden, um die Stirnfläche 1 auszubilden. Alternativ ist es selbstverständlich möglich, die axiale Bewegung axiale Bewegung normal zur Ebene der Kunststofffolie 31 und die radiale Verschiebung der Teilstempel 33 nach außen miteinander zu kombinieren, sodass eine Überlagerung der beiden Bewegungen der Teilstempel 33 vorliegt.

Sofern eine rechteckige oder quadratische Stirnfläche 1 erstellt werden soll, ist es ausreichend, wenn die einzelnen Teilstempel 33 jeweils zu einem auszubildenden Eck der Stirnfläche 1 hin geführt werden, wie dies in **Fig. 7a** dargestellt ist. Sofern jedoch eine kreisrunde Stirnfläche 1 erstellt werden soll, können die Teilstempel 33 zusätzlich noch, wie in **Fig. 7b** dargestellt, in Rotationsbewegung um die gemeinsame Achse X versetzt werden. Die Rotationsbewegung wird von der radial nach außen gerichteten Bewegung der Teilstempel 33 überlagert. Die einzelnen Teilstempel 33 bewegen sich somit entlang eines spiralförmigen Bewegungspfads, wobei jeweils die kreiszylindrischen Mantelflächen der Teilstempel 33 jeweils mit der Kunststofffolie 31 in Kontakt stehen.

Wie bereits zuvor im Zusammenhang mit der Ausbildung von rechteckigen oder quadratischen Stirnflächen 1 beschrieben, ist auch eine kombinierte axiale und radiale Bewegung der Teilstempel 33 bei der Ausbildung von kreisförmigen Stirnflächen 1 möglich. In diesem Fall vollführen die Teilstempel 33 eine Bewegung entlang eines spiralförmigen Bewegungspfads, der schraubenförmig und sich nach unten hin erweiternd spiralförmig verläuft und eine Überlagerung aus einer axialen und einer radialen Bewegung sowie einer Rotationsbewegung um die Achse X darstellt.

Wie in **Fig. 7** dargestellt, weist ist ein Volumen 39 ausgebildet, das gegenüber einer gedachten Fortsetzung der Oberkante der Kunststofffolie 31 eine Höhe $h_1$ aufweist. Diese Höhe wird durch die Vorschublänge der einzelnen Teilstempel 33 axial in Richtung der Achse X festgelegt. Durch die Vorschubgeschwindigkeit der Teilstempel 33 sowie durch die Festlegung der Temperatur der Teilstempel 33 sowie der Kunststofffolie 31 in axialer Richtung wird die Dicke der das Volumen 39 seitlich begrenzenden Mantelwand 2 festgelegt, wobei insbesondere die Dicke in dem Bereich, in dem die Kunststofffolie 31 in die Mantelwand 2 übergeht besonders stark von der jeweiligen Vorschubgeschwindigkeit sowie der jeweiligen Temperatur abhängig ist.

Die Dicke der Mantelwand 2 kann durch die Auswahl der Temperatur der Kunststofffolie 31 sowie der Teilstempel 33 sowie durch die Vorschubgeschwindigkeit der Teilstempel 33 in axialer Richtung festgelegt werden. Die Dicke $d_1$ der Mantelwand 2 im ausgebildeten Mantelwandabschnitt 7 kann dabei kontinuierlich untersucht werden, wobei für den Fall, dass die Dicke $d_1$ zu gering ist, die Vorschubgeschwindigkeit der Teilstempel 33 in axialer Richtung verringert wird oder die Temperatur der Kunststofffolie 31 oder der Teilstempel 33 verringert wird. Ist die Dicke $d_1$ hingegen zu groß, wird die Vorschubgeschwindigkeit der Teilstempel 33 in axialer Richtung erhöht wird oder die Temperatur der Kunststofffolie 31 oder der Teilstempel 33 erhöht.

Im vorliegenden Ausführungsbeispiel wird eine Kunststofffolie 31 mit einer Dicke von 0,4 mm verwendet, die bei der Verarbeitung auf 40 °C erhitzt wurde. Die Teilstempel 33 haben eine Temperatur von 45°C und bilden ein Volumen 39 mit einer Höhe $h_1$ von 4 mm aus. Anschließend werden die Teilstempel 33, wie in **Fig. 7a** dargestellt, radial nach außen bewegt, wobei zunächst eine rechteckige Stirnfläche mit einer Seitenlänge von etwa 4 mm ausgebildet wird. In einem weiteren, in **Fig. 7b** dargestellten Schritt werden die Teilstempel 33 in eine Rotationsbewegung versetzt, wobei die Teilstempel um die Achse X rotieren. Die kreiszylindrischen Mantelflächen der Teilstempel 33 liegen dabei an der Innenwand der ausgebildeten Mantelwand an.

In **Fig. 8** und **Fig. 9** ist die Ausbildung weiterer Mantelwandabschnitte 8, 9 der Abdeckkappe 2 mit unterschiedlicher Dicke $d_2$, $d_3$ dargestellt. Anstelle der Teilstempel 33 wird in den folgenden Schritten ein weiterer Stempel 36 oder mehrere weitere Stempel verwendet, dessen Außenform der Form der jeweiligen Abdeckkappe 10 entspricht.

Wie in **Fig. 8** dargestellt, wird der weitere Stempel 36 in das ausgebildete Volumen 39 vorgeschoben, wobei die jeweilige Wanddicke $d_2$ des in diesem Verfahrensschritt ausgebildeten Bereichs der Mantelwand 2, im Folgenden Zwischenabschnitt 8 genannt, dünner ist als die Wanddicke $d_1$ des Bereichs der Mantelwand 2, im Folgenden erster Verdickungsabschnitt 7 genannt. Die Dicke $d_2$ der Mantelwand 2 im Zwischenabschnitt 8 wird, wie bereits bei der Erstellung des ersten Verdickungsabschnitts 7 ausgeführt, durch die Vorschubgeschwindigkeit des weiteren Stempels 36 sowie durch die Temperatur des weiteren Stempels 36 sowie die Temperatur der Kunststofffolie 31 bestimmt.

Die Außenform des aus der Kunststofffolie 31 ausgebildeten Teils der Abdeckkappe 10, insbesondere des Zwischenabschnitts 8, wird durch eine nicht dargestellte zweite Gegenform festgelegt, die an der äußeren Mantelwand 2 sowie an der Stirnwand 1 der aus der Kunststofffolie 31 ausgebildeten Abdeckkappe 10 anliegt.

Im vorliegenden Ausführungsbeispiel bleibt die Kunststofffolie 31 bei ihrer Verformung auf einer Temperatur von etwa 40°C bis 50°C, anschließend wird der weitere Stempel 36 in die zweite Gegenform eingebracht. Die zweite Gegenform ist auf eine Temperatur von etwa 40°C bis 50°C erhitzt. Der Zwischenbereich zwischen dem weiteren Stempel 36 und der zweiten Gegenform entspricht der gewünschten Form der Abdeckkappe 10 im ersten Verdickungsabschnitt 7 sowie im Zwischenabschnitt 8, im vorliegenden Ausführungsbeispiel ist die Wanddicke im Zwischenabschnitt 8 geringer als im ersten Verdickungsabschnitt 7.

Anschließend wird, wie in **Fig. 9** dargestellt, der weitere Stempel 36 weiter vorgeschoben, wobei ein zweiter Verdickungsabschnitt 9 ausgebildet wird, dessen Dicke $d_3$ größer ist als die Dicke $d_2$ im Zwischenabschnitt.

Die Außenform des aus der Kunststofffolie 31 ausgebildeten Abschnitts der Abdeckkappe 10, insbesondere des an den Zwischenabschnitt 8 anschließenden zweiten Verdickungsabschnitts 9, wird durch eine nicht dargestellte dritte Gegenform festgelegt, die an der äußeren Mantelwand 2 sowie an der Stirnwand 1 der aus der Kunststofffolie 31 ausgebildeten Abdeckkappe 10 anliegt.

Im vorliegenden Ausführungsbeispiel bleibt die Kunststofffolie 31 bei ihrer Verformung auf einer Temperatur von etwa 40°C bis 50°C, anschließend wird der weitere Stempel 36 in die zweite Gegenform eingebracht. Die dritte Gegenform ist auf eine Temperatur von etwa 40°C bis 50°C erhitzt. Der Zwischenbereich zwischen dem weiteren Stempel 36 und der dritten Gegenform entspricht der gewünschten Form der Abdeckkappe 10 im ersten Verdickungsabschnitt 7, im Zwischenabschnitt 8, sowie im zweiten Verdickungsabschnitt.

**Fig. 10** zeigt die Fertigstellung der Abdeckkappe durch Abtrennen von der Kunststofffolie. Nach der Ausformung und Profilierung der gesamten Mantelwand 2 ist die Mantelwand noch mit der Kunststofffolie 31 verbunden. In einem abschließenden Schritt wird die Kunststofffolie 31 entlang einer vorgegebenen Schnittlinie 37 abgetrennt, im vorliegenden Ausführungsbeispiel wird die Kunststofffolie 31 entlang der Mantelwand gestanzt. Dadurch wird die Abdeckkappe 10 vom Rest der Kunststofffolie 31 abgetrennt.

Im vorliegenden Ausführungsbeispiel verläuft die Schnittlinie 37 kreisförmig und koaxial um die hohlkegelstumpfförmige Mantelfläche 2. Durch das Abschneiden entlang der Schnittlinie 37 wird eine Grundwand 3 der Abdeckkappe 10 ausgebildet, die parallel zur Stirnfläche radial von dem der Stirnfläche 1 fernen Ende der Mantelfläche 2 nach außen verläuft. Alternativ besteht auch die Möglichkeit, durch einen weiteren Verformungsprozess die Grundwand 3 von der Stirnwand 1 weg weisend und von der Mantelfläche 2 radial nach außen abstehend in einem Winkel von bis zu 20° insbesondere von bis zu 10° auszubilden.

Nach dem Ende der Ausformung, entweder vor oder nach dem Abschneiden der Abdeckkappe 10 entlang der Schnittlinie 37 wird die die Abdeckkappe 10 bildende Kunststofffolie 31 für einen vorgegebenen Zeitraum von 3 bis 4 Sekunden auf eine Temperatur von zwischen 50 und 120°C je nach verwendetem Kunststoff, im vorliegenden Fall auf 75 °C erhitzt. Im Zuge dieses Vorgangs wird der die Kunststofffolie 31 bildende Kunststoff durch die Erwärmung umgeformt. Die Kunststofffolie 31 verliert ihre thermoplastischen Eigenschaften. Durch die Wärmeeinwirkung werden die die Kunststofffolie 31 bildenden Molekülketten aufgespalten, wodurch eine größere Durchlässigkeit der Abdeckkappe 10, insbesondere im Bereich der Stirnfläche 1 geschaffen wird. Je länger die Wärmeeinwirkung auf die Kunststofffolie 31 dauert und je intensiver diese ist, insbesondere je höher die gewählte Temperatur ist, desto kürzer werden die die Kunststofffolie 31 bildenden Molekülketten und desto größer wird die Durchlässigkeit der Stirnfläche 1.

Alternativ können anstelle des Erhitzens der Kappe auch Löcher 6 in Form, im vorliegenden Fall Mikrolöcher oder Nanolöcher in der Stirnwand 1, vorzugsweise ausschließlich in der Stirnwand 1, ausgebildet werden, um eine erhöhte Durchlässigkeit für Moleküle einer bestimmten Größe zu erzielen. Die Ausbildung der Mikro- und Nanolöcher kann durch Verwendung eines Laserstrahls sowie durch Perforation durch Einstiche mit einer erhitzten Mikronadel erfolgen.

Besonders vorteilhaft ist es, wenn die Abdeckkappe 10 in dem der Umfangskante 4 fernen Bereich eine sehr hohe Steifigkeit aufweist, die der Stabilität der Abdeckkappe dient. Die Steifigkeit der Abdeckkappe 10 nimmt an der Mantelwand 2 zur Mitte hin zu, wobei die Mantelwand 2 in dem die Stirnwand 1 umgebenden Bereich wiederum eine größere Steifigkeit aufweist. Die Elastizität der Mantelwand 4 ist im Bereich ihrer dünnsten Stelle bzw. im Bereich ihrer Stelle mit der geringsten Steifigkeit am größten.

Vorzugsweise ist die Steifigkeit oder die Elastizität der Mantelwand 4 umfangsmäßig homogen verteilt und variiert lediglich mit dem Abstand zur Stirnwand 1.

**Patentansprüche**

1. Abdeckkappe (10), insbesondere zum Aufsetzen auf ein Hautanalysegerät, umfassend eine von einer Umfangskante (4) begrenzte Stirnwand (1) sowie eine an der Umfangskante (4) der Stirnwand (1) anschließende Mantelwand (2), wobei die Stirnwand (1) gasdurchlässig ausgebildet ist,
**dadurch gekennzeichnet, dass** die Abdeckkappe (10) in dem der Stirnwand (1) abgewandten Ende der Mantelwand (2) Rastausnehmungen (5a) und/oder Rastvorsprünge (5b) zur lösbaren Befestigung an einer Messvorrichtung aufweist, und
dass die Mantelwand (2) im Nahebereich der Stirnwand (1) einen, insbesondere an die Umfangskante (4) anschließenden, ersten Verdickungsabschnitt (7) aufweist, in dem die Dicke ($d_1$) der Mantelwand (2) die Dicke ($d_S$) der Stirnwand (1) übersteigt,
dass die Mantelwand (2) einen an den ersten Verdickungsabschnitt (7) anschließenden Zwischenabschnitt (8) aufweist, wobei die Dicke ($d_2$) der Mantelwand (2) im Zwischenabschnitt (8) geringer ist als die Dicke ($d_1$) der Mantelwand (2) im ersten Verdickungsabschnitt (7),
dass die Mantelwand (2) einen an den Zwischenabschnitt (8) anschließenden und bis zu dem der Stirnwand (1) fernen Ende der Mantelwand (2) reichenden zweiten Verdickungsabschnitt (9) aufweist, und
dass die Dicke ($d_3$) der Mantelwand (2) im zweiten Verdickungsabschnitt (9) größer ist als die Dicke ($d_2$) der Mantelwand (2) im Zwischenabschnitt (8).

2. Abdeckkappe nach Anspruch 1, **dadurch gekennzeichnet, dass** die Stirnwand (1) an zumindest einer Stelle eine Dicke ($d_S$) im Bereich von weniger als 100 $\mu$m, vorzugsweise zwischen 0,1 $\mu$m und 20 $\mu$m, insbesondere zwischen 5 $\mu$m und 10 $\mu$m, aufweist, wobei die Stirnwand (1) insbesondere eine über ihren Verlauf konstante Dicke ($d_S$) aufweist, und/oder
dass die Mantelwand (2) im Bereich der Umfangskante (4) der Stirnwand (1) in einem Winkel ($\alpha$) von zwischen 20° und 95°, insbesondere zwischen 60° und 80°, zur Stirnwand (1) nach außen absteht, wobei sich vorzugsweise die Mantelwand (2), insbesondere konisch, erweitert, und/oder
dass insbesondere die einzelnen Rastausnehmungen (5a) und/oder Rastvorsprünge (5b) jeweils denselben Normalabstand zur Stirnwand (1) aufweisen und/oder in Umfangsrichtung der Mantelwand (2) gleichmäßig verteilt sind, und/oder wobei die Gesamtzahl der Rastausnehmungen (5a) und/oder Rastvorsprünge (5b) zwei oder vier beträgt, und/oder
dass insbesondere die Rastausnehmungen (5a) und/oder Rastvorsprünge (5b) in einem Winkel von ($\beta_1$, $\beta_2$) 5° bis 40° und/oder höchstens 2 mm, insbesondere höchstens 0,3 mm von der Mantelwand (2) abstehen oder in diese eindringen, und/oder
dass die Stirnwand (1) strahlungs-, dampf-, feuchtigkeits-, partikel-, und/oder lichtdurchlässig ausgebildet ist und/oder eine Anzahl von, insbesondere dampfdurchlässigen oder gasdurchlässigen, Mikrolöchern (6) aufweist, und/oder
dass die Dicke ($d_2$) der Mantelwand (2) von der Stirnwand (1) zu dem von der Stirnwand (1) fernen Ende der Mantelwand (2) hin, insbesondere zumindest über einen Teilbereich der Mantelwand (2), vorzugsweise kontinuierlich, zunimmt, und/oder
dass die Stirnwand (1) kreisförmig ausgebildet ist und insbesondere einen Durchmesser von 1 mm bis 50 mm aufweist, oder dass die Stirnwand (1) quadratisch oder rechteckig ausgebildet ist und wobei die Seitenkanten der Stirnwand (1) eine Länge von zwischen 2 mm und 40 mm aufweisen.

3. Abdeckkappe (10) nach Anspruch 1 oder 2, **gekennzeichnet durch** eine Grundwand (3), die die Mantelwand (2) an dem von der Stirnwand (1) abgewandten Ende, insbesondere einstückig, fortsetzt, und die vorzugsweise parallel oder in einem Winkel von höchstens 10° zur Stirnwand (1) von der Mantelwand (2) nach außen, insbesondere mit ansteigendem Normalabstand zur Stirnwand (1), verläuft, wobei die Grundwand (3) vorzugsweise kreisringförmig ausgebildet ist und insbesondere einen Außenradius zwischen 2 mm und 50 mm und/oder einen Innenradius zwischen 1 mm und 45 mm aufweist und/oder eine Kreisringbreite von 0,5 mm bis 35 mm aufweist.

4. Abdeckkappe (10) nach einem der vorangehenden Ansprüche, **dadurch gekennzeichnet, dass** die Abdeckkappe (10) materialeinheitlich und/oder einstückig ausgebildet ist, und insbesondere aus thermoplastischem oder plastisch verformten oder verformbaren Kunststoff, vorzugsweise aus einem der folgenden Materialien oder einer Mischung hiervon, besteht:

- PETG (Polyethylenterephthalat Glycol),
- PP (Polypropylen),
- PE (Polyethylen),
- PC (Polycarbonat),
- PVC (Polyvinylchlorid),
- PS (Polystyrol),
- ABS (Acrylonitrile butadiene styrene),
- HDPE (High Density Polyethylene),
- LDPE (Low Density Polyethylene),
- PET (Polyethylene terephthalate),
- PMMA (Polymethyl methacrylate),
- ECOTERM S 900 T1,
- PETG / Copolyester 67639,

wobei das Material der Abdeckkappe (10), insbesondere zusätzlich, einen oder mehrere der folgenden Bestandteile enthält:

- Additive,
- Stabilisatoren,
- Farbmittel,
- Füllstoffe,
- Verstärkungsstoffe,

wobei insbesondere das Material der Abdeckkappe (10) einen Farbstoff aufweist und die Stirnwand (1) für bestimmte, insbesondere sichtbare, Wellenlängenanteile undurchsichtig oder absorbierend ausgebildet ist.

5. Abdeckkappe (10) nach einem der vorangehenden Ansprüche, **dadurch gekennzeichnet, dass** die Dicke der Mantelwand (2), insbesondere in axialer oder radialer Richtung, einen über die Mantelwand (2) kontinuierlichen, knick- und kantenfreien Verlauf aufweist, und/oder als Dicke ($d_1$, $d_2$, $d_3$) die jeweils mittlere Dicke im jeweiligen Abschnitt (7, 8, 9) der Mantelwand (2) gilt, und/oder dass die Mantelwand (2), insbesondere mit Ausnahme des Bereichs der Rastvorsprünge (5a) und/oder Rastausnehmungen (5b), rotationssymmetrisch ausgebildet ist, und/oder dass die Dicken ($d_1$, $d_2$, $d_3$) des ersten Verdickungsabschnitts (7), des Zwischenabschnitts (8) und des zweiten Verdickungsabschnitts (9) zueinander im folgenden Verhältnis stehen:

$$2 < d_1 : d2 < 5 \text{ und/oder } 2 < d_3 : d_2 < 5 \text{ und/oder } 0,8 < d_1 : d_3 < 1,25$$

und/oder dass die Dicken ($d_1$, $d_2$, $d_3$) des ersten Verdickungsabschnitts (7), des Zwischenabschnitts (8) und des zweiten Verdickungsabschnitts (9) wie folgt festgelegt sind:

$$150 \ \mu m < d_1 < 250 \ \mu m \text{ und/oder } 50 \ \mu m < d_2 < 100 \ \mu m \text{ und/oder } 150 \ \mu m < d_3 < 250 \ \mu m, \text{ und/oder}$$

dass die Höhe ($h_1$) des ersten Verdickungsabschnitts (7), die Höhe ($h_2$) des Zwischenabschnitts (8) sowie die Höhe ($h_3$) des zweiten Verdickungsabschnitts (9) in folgendem Verhältnis stehen:

$$0,2 < h_1 : h_2 < 0,6 \text{ und/oder } 0,8 < h_3 : h_2 < 1,25 \text{ und/oder } 0,2 < h_1 : h_3 < 0,6 \text{ und/oder}$$

- dass die Mantelwand (2) eine Höhe von 2 mm bis 80 mm aufweist und/oder
- dass das Verhältnis zwischen der Höhe und der maximalen Abmessung, insbesondere der Durchmesser oder die Diagonale, der Stirnwand (1) zwischen 0,01 und 55 liegt.

6. Messgerät (20), insbesondere zur Hautanalyse, das mit einer Abdeckkappe (10) abdeckbar ist, wobei die Abdeckkappe (10) Rastausnehmungen (5a) und/oder Rastvorsprüngen (5b) aufweist, wobei die Abdeckkappe (10) vorzugsweise nach einem der Ansprüche 1 bis 3 ausgebildet ist,

wobei das Messgerät (20) ein Gehäuse (26) und einen darin angeordneten Sensor (23) aufweist, wobei das Gehäuse (26) einen konisch zulaufenden Gehäuseteil (21) mit einer vorderen Stirnfläche (22) aufweist, an dem eine Anzahl von Rastausnehmungen (23a) und/oder Rastvorsprüngen (25b) zum Verrasten mit den Rastausnehmungen (5a) und/oder Rastvorsprüngen (5b) der Abdeckkappe (10) angeordnet ist,

insbesondere derart, dass im eingerasteten Zustand die Stirnwand (1) der Abdeckkappe (10) an der vorderen Stirnfläche (22) anliegt,

- wobei das Messgerät (20) ein Auswurfelement aufweist, das in dem der vorderen Stirnwand (22) entfernten Ende des konisch zulaufenden Gehäuseteils (21) angeordnet ist und das von der vorderen Stirnwand (22) weiter entfernt ist als die Rastausnehmungen (25a) und/oder Rastvorsprüngen (25b),
- wobei das Auswurfelement (24) zum Schieben einer eingerasteten Abdeckkappe (10) durch Druckbeaufschlagung der Abdeckkappe (10) in Richtung der vorderen Stirnwand (22) ausgebildet ist und/oder
- wobei das Auswurfelement auf dem Gehäuse in Richtung der vorderen Stirnwand (22) verschiebbar gelagert ist,
- wobei an das Auswurfelement (24) ein Hebelfortsatz (27) anschließt oder von dem Auswurfelement (24) ein Hebelfortsatz abgeht oder das Auswurfelement (24) mit einem Hebelfortsatz (27) verbunden ist,
- wobei der Hebelfortsatz (27) in seinem Mittenbereich schwenkbar mit dem Gehäuse (26) verbunden ist, sodass sich ein zweiarmiger Hebel ergibt, an dessen einen Ende das Auswurfelement (24) angeordnet ist,
- wobei der am Gehäuse angelenkte Hebelfortsatz (27) bei seinem Verschwenken, das Auswurfelement (24) in Richtung der vorderen Stirnwand (22) drückt,

**dadurch gekennzeichnet, dass** das Auswurfelement (24) ringförmig, insbesondere kreisringzylinderförmig, ausgebildet ist und den konisch zulaufenden Gehäuseteil (21) umgibt.

7. Messgerät (20) nach Anspruch 6, **dadurch gekennzeichnet, dass** der am Gehäuse angelenkte Hebelfortsatz (27) bei seinem Verschwenken durch Kraftbeaufschlagung des Betätigungselements (28), das Auswurfelement (24) in Richtung der vorderen Stirnwand (22) drückt.

8. Messgerät (20) nach einem der Ansprüche 6 oder 7, **dadurch gekennzeichnet, dass** das Auswurfelement (24) an einem Ende des zweiarmigen Hebels angeordnet ist und am anderen Ende des zweiarmigen Hebels ein Betätigungselement (28) vorgesehen ist, wobei insbesondere

das Auswurfelement (24) und/oder der Hebelfortsatz (27) durch ein Federelement (29) mit einer Kraft oder Vorspannung beaufschlagt ist, die das Auswurfelement (24) von der vorderen Stirnwand (22) weg drückt oder weg zieht.

9. Messgerät (20) nach einem der Ansprüche 6 bis 8, **dadurch gekennzeichnet, dass**
die einzelnen Rastausnehmungen (25a) und/oder Rastvorsprünge (25b) jeweils denselben Normalabstand zur Stirnfläche (22) aufweisen und/oder in Umfangsrichtung der Mantelwand (2) gleichmäßig verteilt sind, und/oder die Gesamtzahl der Rastausnehmungen (25a) und/oder Rastvorsprünge (25b) zwei oder vier beträgt, und/oder
dass die Rastausnehmungen (25a) und/oder Rastvorsprünge (25b) in einem Winkel von 10° bis 30° und/oder höchstens 1 mm, insbesondere höchstens 0,5 mm, von der Mantelwand (2) abstehen und/oder
dass zwei Rastausnehmungen (25a) und/oder Rastvorsprünge (25b) vorgesehen sind, die denselben Abstand zu einem Punkt, in dem Bereich aufweisen, an dem der Hebelfortsatz (27) an das Auswurfelement (24) anschließt oder von diesem abgeht.

10. Verfahren zur Herstellung einer Abdeckkappe (10), insbesondere nach einem der Ansprüche 1 bis 5,

a) dass eine Kunststofffolie (31), insbesondere eine Folie bestehend aus PETG (Polyethylenterephthalat Glycol), als Ausgangsmaterial für einen Tiefziehprozess herangezogen wird, die vorzugsweise eine Dicke zwischen 0,04 mm und 0,5 mm aufweist,
b) dass ein Stempel (32) mit einer Anzahl von separat beweglichen, aneinander anliegenden Teilstempeln (33) auf die Kunststofffolie (31) gepresst wird, wobei die einzelnen Teilstempel (33) normal zur Ebene der Kunststofffolie (31) bewegt werden und wobei die Kunststofffolie (31) durch den Druck der Teilstempel (33) in eine Gegenform gedrückt wird und zu einer Kappenform verformt wird, **dadurch gekennzeichnet,**
c) **dass** die einzelnen Teilstempel (33) des Stempels (32) während oder nach Schritt b) zueinander beabstandet werden, wobei jeder der Teilstempel (33) von der Position des Stempels (32) aus radial nach außen bewegt wird, wodurch in der Kunststofffolie (31) eine Stirnwand (1), insbesondere mit einer Dicke im Bereich von weniger als 100 $\mu$m, vorzugsweise zwischen 3 $\mu$m und 20 $\mu$m, insbesondere zwischen 5 $\mu$m und 10 $\mu$m, ausgebildet wird.

11. Verfahren nach Anspruch 10, **dadurch gekennzeichnet, dass** anschließend an Schritt c) die Teilstempel (33) aus der ausgebildeten Kappenform entfernt und ein weiterer Stempel (36) mit einer ebenen Stirnfläche (35) und einem sich erweiternden Stempelkörper, in das durch die Teilstempel (33) ausgeformte Volumen (39) der Kunststofffolie (31) eingebracht wird, wobei die Kunststofffolie (31) zwischen dem weiteren Stempel (36) und einer zweiten Gegenform in eine Kappenform gebracht wird, wobei vorzugsweise die Kunststofffolie (31), der weitere Stempel (36) oder die zweite Gegenform nach dem Erhitzen der Kunststofffolie (31) auf eine Temperatur zwischen 50°C und 90°,C vorgeschoben wird.

12. Verfahren nach Anspruch 10 oder 11, **dadurch gekennzeichnet, dass** zur Einstellung der jeweiligen Dicke ($d_1$, $d_2$, $d_3$) der von der Stirnwand (1) abgehenden Mantelwand (2) die Vorschubgeschwindigkeit (v) und/oder Temperatur der Teilstempel (33), des weiteren Stempels (36) und/oder die Temperatur der Kunststofffolie (31) und/oder die Temperatur der ersten oder zweiten Gegenform eingestellt wird,
wobei zur Verringerung der Wandstärke im Randbereich (36) der durch den weiteren Stempel gebildeten Ausnehmung eine Erhöhung der genannten Temperaturen sowie eine Erhöhung der Vorschubgeschwindigkeit vorgenommen wird, und
wobei zur Erhöhung der Wandstärke im Randbereich (36) der durch den weiteren Stempel gebildeten Ausnehmung eine Verringerung der genannten Temperaturen sowie eine Verringerung der Vorschubgeschwindigkeit vorgenommen wird.

13. Verfahren nach einem der Ansprüche 10 bis 12, **dadurch gekennzeichnet, dass** die Temperatur des weiteren Stempels (36) und/oder der Kunststofffolie (31) sowie die Vorschubgeschwindigkeit des weiteren Stempels (36) derart eingestellt werden,
dass während eines ersten Vorschubzeitraums des weiteren Stempels (36) ein Abschnitt der Mantelwand (2) mit einer vorgegebenen Dicke ($d_2$), insbesondere von 50 $\mu$m bis 100 $\mu$m, ausgebildet wird, und
dass während eines dem ersten Vorschubzeitraums nachfolgenden zweiten Vorschubzeitraums des weiteren Stempels (36) ein Abschnitt der Mantelwand (2) mit einer vorgegebenen Dicke ($d_3$), insbesondere von 150 $\mu$m und 250 $\mu$m, ausgebildet wird, die dicker ist als die Dicke ($d_2$) der im ersten Vorschubzeitraum erstellten Mantelwand (2), wobei insbesondere die Wanddicke ($d_2$) während des ersten Vorschubzeitraums des weiteren Stempels (36) auf einen Wert eingestellt wird, der dem Doppelten bis Fünffachen der Wanddicke ($d_1$) desjenigen Mantelbereichs entspricht, der bei der Ausformung der Stirnwand (1) durch das Einbringen der Teilstempel (33) ausgeformt wurde.

14. Verfahren nach einem der Ansprüche 10 bis 13, **dadurch gekennzeichnet, dass** die Kunststofffolie (31) aus thermoplastischem oder plastisch verformten oder verformbaren Kunststoff, vorzugsweise aus einem der folgenden Materialien oder einer Mischung hiervon, besteht:

- PETG (Polyethylenterephthalat Glycol),
- PP (Polypropylen),
- PE (Polyethylen),
- PC (Polycarbonat),
- PVC (Polyvinylchlorid),
- PS (Polystyrol),
- ABS (Acrylonitrile butadiene styrene),
- HDPE (High Density Polyethylene),
- LDPE (Low Density Polyethylene),
- PET (Polyethylene terephthalate),
- PMMA (Polymethyl methacrylate),
- ECOTERM S 900 T1,
- PETG / Copolyester 67639,

wobei das Material der Abdeckkappe (10), insbesondere zusätzlich, einen oder mehrere der folgenden Bestandteile enthält:

- Additive,
- Stabilisatoren,
- Farbmittel,
- Füllstoffe,
- Verstärkungsstoffe,

wobei insbesondere das Material der Abdeckkappe (10) einen Farbstoff aufweist und die Stirnwand (1) für bestimmte Wellenlängenanteile undurchsichtig oder absorbierend ausgebildet wird.

**15.** Verfahren nach einem der Ansprüche 10 bis 14, **dadurch gekennzeichnet,**

- **dass** die Teilstempel (33) während ihrer radial von ihrem Zentrum weg gerichteten Bewegung in Schritt c) entlang einer um eine zentrale Achse (X) spiralförmig nach außen verlaufenden und sich aufweitenden Kurve rotierend bewegt werden, und/oder
- **dass** nach der teilweisen oder vollständigen Ausformung der Mantelwand (2) die Teilstempel (33) oder der weitere Stempel (36) zurückgefahren werden und das ausgeformte Volumen (39) mit, insbesondere erhitzter Druckluft, beaufschlagt wird und dass der weitere Stempel (36) anschließend gegebenenfalls in das ausgeformte Volumen (39) zur Durchführung des nächsten Ausformungsschritt eingebracht wird, wobei insbesondere durch Beaufschlagung mit Druckluft Rastausnehmungen (5b) im Innenmantel der Mantelwand (2) ausgeformt werden, und/oder dass nach der Ausformung der Mantelwand (2) die an die Mantelwand (2) anschließende verbleibende Kunststofffolie (31) von der Mantelwand (2) entlang einer vorgegebenen Schnittlinie (37) abgetrennt wird, wobei die Schnittlinie (37) insbesondere so gewählt wird, dass ein Teilabschnitt der an die Mantelwand (2) anschließenden verbleibenden Kunststofffolie (31) innerhalb der Schnittlinie liegt und eine Grundwand (3) ausbildet, und/oder
- **dass** die Kunststofffolie (31), insbesondere nach ihrer Ausformung, für einen vorgegebenen Zeitraum, insbesondere für zumindest 3 Sekunden, auf eine vorgegebene Temperatur zwischen 30°C bis 120°C, insbesondere auf eine Temperatur zwischen 50°C und 90°C, aufgewärmt wird, und/oder

**dass** die Teilstempel (33) und/oder der weitere Stempel (36) und/oder die verwendeten Gegenformen vor der Bearbeitung der Kunststofffolie (31) auf eine Temperatur zwischen 30°C bis 120°C, insbesondere auf eine Temperatur zwischen 50°C und 90°C, erwärmt werden und während der Bearbeitung auf einer solchen Temperatur gehalten werden, und/oder dass, insbesondere mittels eines Lasers oder einer erhitzten Mikronadel oder Nanonadel oder mittels Ätzen, Löcher (6), insbesondere Mikrolöcher oder Nanolöcher (6), in der Stirnwand (1) ausgebildet werden.

### Claims

1. A covering cap (10), in particular for placing on a skin analyzer, comprising an end wall (1) bounded by a circumferential edge (4) as well as a casing wall (2) connecting to the circumferential edge (4) of the end wall (1), wherein the end wall is designed to be gas-permeable, **characterized in that** the covering cap (10) in the end of the casing wall (2) turned away from the end wall (1) has catch recesses (5a) and/or catch projections (5b) for the detachable mounting on a measurement device, and that the casing wall (2) in the vicinity of the end wall (1) has a first thickening section (7) connecting in particular to the circumferential edge (4), in which the thickness ($d_1$) of the casing wall (2) exceeds the thickness ($d_S$) of the end wall (1),
that the casing wall (2) has an intermediate section (8) connecting to the first thickening section (7), wherein the thickness ($d_2$) of the casing wall (2) is less in the intermediate section (8) than the thickness ($d_1$) of the casing wall (2) in the first thickening section (7), that the casing wall (2) has a second thickening section (9) connecting to the intermediate section (8) and extending up to the end of the casing wall (2) remote from the end wall (1), and
that the thickness ($d_3$) of the casing wall (2) in the second thickening section (9) is greater than the thickness ($d_2$) of the casing wall (2) in the intermediate section (8).

2. A covering cap according to Claim 1, **characterized in that** the end wall (1) has in at least one place a thickness ($d_s$) in the range of less than 100 $\mu$m, preferably between 0.1 $\mu$m and 20 $\mu$m, in particular between 5 $\mu$m and 10 $\mu$m, wherein the end wall has in particular a constant thickness ($d_s$) over its course, and/or that the casing wall (2) in the area of the circumferential edge (4) of the end wall (1) protrudes outwards to the end wall (1) at an angle ($\alpha$) of between 20° and 95°, in particular between 60° and 80°, wherein preferably the casing wall (2), expands, in particular conically, and/or

that in particular, the individual catch recesses (5a) and/or catch projections (5b) in each case have the same standard distance to the end wall (1) and/or are evenly distributed in the circumferential direction of the casing wall (2), and/or wherein the total number of catch recesses (5a) and/or catch projections (5b) is two or four, and/or

that in particular the catch recesses (5a) and/or catch projections (5b) protrude at an angle of ($\beta_1$, $\beta_2$) 5° to 40° and/or at most 2 mm, in particular at most 0.3 mm from the casing wall (2) or penetrate into the latter, and /or

that the end wall (1) is designed to be permeable to radiation, vapor, moisture, particles, and/or light and/or has a number of, in particular vapor permeable or gas permeable micro-holes (6), and/or

that the thickness ($d_2$) of the casing wall (2) from the end wall (1) to the end of the casing wall (2) remote from the end wall (1), in particular, at least over a subsection of the casing wall (2), increases, preferably continuously, and/or

that the end wall (1) is designed to be circular and in particular has a diameter of 1 mm to 50 mm, or that the end wall (1) is designed to be square or rectangular and wherein the side edges of the end wall (1) have a length of between 2 mm and 40 mm.

3. A covering cap (10) according to Claim 1 or 2 **characterized by** a base wall (3), which continues the casing wall (2) on the end turned away from the end wall (1), in particular, in one piece, and which preferably runs parallel or at an angle of at most 10° to the end wall (1) from the casing wall (2) outwards, in particular, with increasing standard distance to the end wall (1), wherein the base wall (3) is preferably designed in the shape of a circular ring and in particular has an outer radius between 2 mm and 50 mm and/or an inner radius between 1 mm and 45 mm and/or has a circular ring width of 0.5 mm to 35 mm.

4. A covering cap (10) according to one of the preceding claims, **characterized in that** the covering cap (10) is designed materially uniform and/or in one piece, and in particular consists of thermoplastically or plastically deformed or deformable synthetic material, preferably of one of the following materials or a mixture thereof:

- PETG (polyethylene terephthalate glycol),
- PP (polypropylene),
- PE (polyethylene),
- PC (polycarbonate),
- PVC (polyvinyl chloride),
- PS (polystyrene),
- ABS (acrylonitrile butadiene styrene),
- HDPE (high density polyethylene),
- LDPE (low density polyethylene),
- PET (polyethylene terephthalate),
- PMMA (polymethyl methacrylate),
- ECOERM S 900 T1,
- PETG / copolyester 67639,

wherein the material of the covering cap (10), in particular, additionally contains one or several of the following components:

- additives,
- stabilizers,
- colorants,
- filler materials,
- reinforcing materials,

wherein in particular the material of the covering cap (10) has a colorant and the end wall (1) is designed opaquely or absorbingly for certain, particularly visible, wave length components.

5. A covering cap (10) according to one of the preceding claims, **characterized in that** the thickness of the casing wall (2), in particular in the axial or radial direction, has a continuous, kink- and edge-free course across the casing wall (2), and/or

the in each case average thickness in the respective section (7, 8, 9) of the casing wall (2) is regarded as thickness ($d_1$, $d_2$, $d_3$), and/or

that the casing wall (2), in particular with the exception of the area of the catch projections (5a) and/or catch recesses (5b), is designed rotationally symmetrical, and/or that the thicknesses ($d_1$, $d_2$, $d_3$) of the first thickening section (7), the intermediate section (8) and the second thickening section (9) are in the following ratio to each other:

$2 < d_1 : d_2 < 5$ and/or $2 < d_3 : d_2 < 5$ and/or $0.8 < d_1 : d_3 < 1.25$ and/or that the thicknesses ($d_1$, $d_2$, $d_3$) of the first thickness section (7), the intermediate section (8) and the second thickness section (9) are defined as follows:

$150\mu m < d_1 < 250 \mu m$ and/or $50 \mu m < d_2 < 100 \mu m$ and/or $150 \mu m < d_3 < 250 \mu m$, and/or that the height ($h_1$) of the first thickness section (7), the height ($h_2$) of the intermediate section (8) as well as the height ($h_3$) of the second thickness section (9) are in the following ratio:

$2 < h_1\ h_2 < 0.6$ and/or $0.8 < h_3 : h_2 < 1.25$ and/or $0.2 < h_1 : h_3 < 0.6$ and/or

- that the casing wall (2) has a height of 2 mm to 80 mm and/or
- that the ratio between the height and the maximum dimension, in particular, the diameter or the diagonals, of the end wall (1) is between 0.01 and 55.

6. A measuring device (20), in particular for skin analysis, which can be covered with a covering cap (10), wherein the covering cap (10) has catch recesses (5a) and/or catch projections (5b), wherein the covering cap (10) is designed preferably according to one of Claims 1 to 3,
wherein the measuring device (20) has a housing (26) and a sensor (23) arranged therein, wherein the housing (26) has a conically tapering housing part (21) with a front end face (22), on which a number of catch recesses (23a) and/or catch projections (25b) are arranged for catching with the catch recesses (5a) and/or catch projections (5b) of the covering cap (10),
in particular in such a way that in the latched state the end wall (1) of the covering cap (10) abuts against the front end face (22),

- wherein the measuring device (20) has an ejection element, which is arranged in the end of the conically tapering housing part (21) remote from the front end wall (22) and which is further away from the front end wall (22) than the catch recesses (25a) and/or catch projections (25b),
- wherein the ejection element (24) is designed for pushing a latched covering cap (10) through pressurization of the covering cap (10) in the direction of the front end wall (22) and/or
- wherein the ejection element is mounted in a movable manner on the housing in the direction of the front end wall (22),
- wherein a lever extension (27) connects to the ejection element (24) or a lever extension branches off from the ejection element (24) or the ejection element (24) is connected with a lever extension (27),
- wherein the lever extension (27) is connected in its middle area pivoted with the housing (26), so that a two-armed lever results, on one end of which the ejection element (24) is arranged,
- wherein the lever extension (27) hinged on the housing during its pivoting motion pushes the ejection element (24) in the direction of the front end wall (22), **characterized in that** the ejection element (24) is designed ring-shaped, in particular circular ring cylinder-shaped and surrounds the conically tapering housing part (21).

7. A measuring device (20) according to Claim 6, **characterized in that** the lever extension (27) hinged on the housing pushes the ejection element (24) in the direction of the front end wall (22) during its pivoting motion through application of force of the actuating element (28).

8. A measuring device (20) according to one of Claims 6 or 7, **characterized in that** the ejection element (24) is arranged on an end of the two-armed lever and on the other end of the two-armed lever an actuating element (28) is provided,
wherein in particular
the ejection element (24) and/or the lever extension (27) is subjected to force or pretension by a spring element (29), which pushes or pulls the ejection element (24) away from the front end wall (22).

9. A measuring device (20) according to one of Claims 6 to 8, **characterized in that** the individual catch recesses (25a) and/or catch projections (25b) in each case have the same standard distance to the end face (22) and/or are evenly distributed in the circumferential direction of the casing wall (2), and/or the total number of the catch recesses (25a) and/or catch projections (25b) is two or four, and/or
that the catch recesses (25a) and/or catch projections (25b) protrude from the casing wall (2) at an angle of 10° to 30° and/or at most 1 mm, in particular at most 0.5 mm and/or
that two catch recesses (25a) and/or catch projections (25b) are provided, which have the same distance to a point, in the area, on which the lever extension (27) connects to the ejection element (24) or branches off from the latter.

10. A method for manufacturing a covering cap (10), in particular according to one of Claims 1 to 5,

   a) that a plastic film (31), in particular a film consisting of PETG (polyethylene terephthalate glycol) is used as raw material for a thermo-forming process, which preferably has a thickness between 0.04 mm and 0.5 mm,
   b) that a punch (32) with a number of separately movable partial punches (33) abutting each other is pressed onto the plastic film (31), wherein the individual partial punches are moved normal to the plane of the plastic film (31) and wherein the plastic film (31) is pressed by the pressure of the partial punch (33) into a counter-form and is deformed into a cap form, **characterized in**
   c) **that** the individual partial punches (33) of the punch (32) are spaced apart from each other during or after step b), wherein each of the partial punches (33) is moved radially outwards from the position of the punch (32), whereby an end wall (1) is formed in the plastic film (31), in particular with a thickness in the range of less than 100 $\mu$m, preferably between 3 $\mu$m and 20 $\mu$m, in particular between 5 $\mu$m and 10 $\mu$m.

11. A method according to Claim 10, **characterized in that** subsequent to step c) the partial punch (33) is removed from the cap form formed and a further punch (36) with a level end face (35) and an expanding punch body is introduced into the volume (39) of the plastic film (31) formed by the partial punch (33), wherein the plastic film (31) is brought into a cap form between the further punch (36) and a second counter-form, wherein preferably the plastic film (31), the further punch (36) or the second counter-form is fed forward after the heating of the plastic film (31) to a temperature between 50°C and 90°C.

12. A method according to Claim 10 or 11, **characterized in that** to set the respective thickness ($d_1$, $d_2$, $d_3$) of the casing wall (2) branching off from the end wall (1) the feed velocity (v) and/or temperature of the partial punches (33), of the further punch (36) and/or the temperature of the plastic film (31) and/or the temperature of the first or second counter-form is set,
   wherein to reduce the wall thickness in the edge region (36) of the recess formed by the further punch an increase of the temperatures mentioned as well as an increase of the feed velocity is made, and
   wherein to increase the wall thickness in the edge region (36) of the recess formed by the further punch a reduction of the temperatures mentioned as well as a reduction of the feed velocity is made.

13. A method according to one of Claims 10 to 12, **characterized in that** the temperature of the further punch (36) and/or of the plastic film (31) as well as the feed velocity of the further punch (36) are set in such a way
   that during a first feed time period of the further punch (36) a section of the casing wall (2) is formed with a preset thickness ($d_2$), in particular of 50 $\mu$m to 100 $\mu$m, and
   that during a second feed time period of the further punch (36) following the first feed time period a section of the casing wall (2) is formed with a preset thickness ($d_3$), in particular of 150 $\mu$m and 250$\mu$m, which is thicker than the thickness ($d_2$) of the casing wall (2) created in the first feed time period, wherein in particular the wall thickness ($d_2$) during the first feed time period of the further punch (36) is set to a value, which corresponds to the doubling to quintupling of the wall thickness ($d_1$) of that casing area, which was formed during the shaping of the end wall (1) by the introduction of the partial punches (33).

14. A method according to one of Claims 10 to 13, **characterized in that** the plastic film (31) consists of thermoplastically or plastically deformed or deformable synthetic material, preferably of one of the following materials or a mixture thereof:

   - PETG (polyethylene terephthalate glycol),
   - PP (polypropylene),
   - PE (polyethylene),
   - PC (polycarbonate),
   - PVC (polyvinyl chloride),
   - PS (polystyrene),
   - ABS (acrylonitrile butadiene styrene),
   - HDPE (high density polyethylene),
   - LDPE (low density polyethylene),
   - PET (polyethylene terephthalate),
   - PMMA (polymethyl methacrylate),
   - ECOERM S 900 T1,

- PETG / copolyester 67639,

wherein the material of the covering cap (10), in particular, additionally contains one or several of the following components:

- additives,
- stabilizers,
- colorants,
- filler materials,
- reinforcing materials,

wherein in particular the material of the covering cap (10) has a colorant and the end wall (1) is designed opaquely or absorbingly for certain, particularly visible, wave length components.

15. A method according to one of Claims 10 to 14, **characterized in**

- **that** the partial punches (33) during their radial movement directed away from the center in step c) are moved rotating along a curve running about a central axis (X) spirally outwards and expanding, and/or
- **that** after the partial or complete formation of the casing wall (2) the partial punches (33) or the further punch (36) are driven back and the formed volume (39) is subjected in particular to heated compressed air and that the further punch (36) is subsequently, if necessary, introduced into the formed volume (39) to implement the next forming step, wherein in particular through applying compressed air catch recesses (5b) are formed in the inner casing of the casing wall (2), and/or that after the formation of the casing wall (2) the remaining plastic film (31) connecting to the casing wall (2) is separated from the casing wall (2) along a preset cutting line (37), wherein the cutting line (37) is selected in particular so that a subsection of the remaining plastic film (31) connecting to the casing wall (2) lies inside the cutting line and forms a base wall (3), and/or
- **that** the plastic film (31), in particular after its formation is heated for a preset time period, in particular for at least 3 seconds, to a preset temperature between 30°C to 120°C, in particular to a temperature between 50°C and 90°C, and/or

**that** the partial punches (33) and/or the further punch (36) and/or the counter-forms used before the processing of the plastic film (31) are heated to a temperature between 30°C to 120°C, in particular to a temperature between 50°C and 90°C and during the processing are maintained at such a temperature, and/or that, in particular holes (6), in particular micro-holes or nano-holes (6) are formed in the end wall (1) by means of a laser or a heated micro-needle or nano-needle or by means of etching.

## Revendications

1. Capuchon de protection (10), plus particulièrement destiné à être posé sur un appareil d'analyse de la peau, comprenant une paroi frontale (1) limitée par un bord périphérique (4) ainsi qu'une paroi d'enveloppe (2) prolongeant le bord périphérique (4) de la paroi frontale (1),
la paroi frontale (1) étant conçue de façon à être perméable aux gaz,
**caractérisé en ce que** le capuchon de protection (10) comprend, dans l'extrémité de la paroi d'enveloppe (2) opposée à la paroi frontale (1), des évidements d'encliquetage (5a) et/ou des saillies d'encliquetage (5b) pour la fixation amovible sur un dispositif de mesure et
**en ce que** la paroi d'enveloppe (2) comprend, à proximité de la paroi frontale (1), une première portion d'épaississement (7) prolongeant plus particulièrement le bord périphérique (4), dans laquelle l'épaisseur ($d_1$) de la paroi d'enveloppe (2) dépasse l'épaisseur ($d_s$) de la paroi frontale (1),
**en ce que** la paroi d'enveloppe (2) comprend une portion intermédiaire (8) prolongeant la première portion d'épaississement (7), l'épaisseur ($d_2$) de la paroi d'enveloppe (2) étant, dans la portion intermédiaire (8), inférieure à l'épaisseur ($d_1$) de la paroi d'enveloppe (2) dans la première portion d'épaississement (7),
**en ce que** la paroi d'enveloppe (2) comprend une deuxième portion d'épaississement (9) prolongeant la portion intermédiaire (8) et atteignant l'extrémité de la paroi d'enveloppe (2) éloignée de la paroi frontale (1) et
**en ce que** l'épaisseur ($d_3$) de la paroi d'enveloppe (2) dans la deuxième portion d'épaississement (9) est supérieure à l'épaisseur ($d_2$) de la paroi d'enveloppe (2) dans la portion intermédiaire (8).

2. Capuchon de protection selon la revendication 1, **caractérisé en ce que** la paroi frontale (1) présente, à au moins un endroit, une épaisseur (ds) de l'ordre de moins de 100 $\mu$m, de préférence entre 0,1 $\mu$m et 20 $\mu$m, plus particulièrement entre 5 $\mu$m et 10 $\mu$m, la paroi frontale (1) présentant plus particulièrement une épaisseur (ds) constante sur toute son extension et/ou

**en ce que** la paroi d'enveloppe (2) forme vers l'extérieur, au niveau du bord périphérique (4) de la paroi frontale (1), un angle ($\alpha$) entre 20° et 95°, plus particulièrement entre 60° et 80°, par rapport à la paroi frontale (1), de préférence la paroi d'enveloppe (2) s'élargissant, plus particulièrement de manière conique et/ou en ce que, plus particulièrement, les différents évidements d'encliquetage (5a) et/ou des saillies d'encliquetage (5b) présentent la même distance normale par rapport à la paroi frontale (1) et/ou sont répartis uniformément dans la direction périphérique de la paroi d'enveloppe (2) et/ou le nombre total d'évidements d'encliquetage (5a) et/ou de saillies d'encliquetage (5b) est deux ou quatre et/ou

**en ce que** plus particulièrement les évidements d'encliquetage (5a) et/ou les saillies d'encliquetage (5b) s'écartent de la paroi d'enveloppe (2) ou pénètrent dans celle-ci avec un angle ($\beta1$, $\beta2$) de 5° à 40° et/ou se trouvent à 2 mm maximum, plus particulièrement à 0,3 mm maximum de celle-ci et/ou

**en ce que** la paroi frontale (1) est conçue de façon à être perméable aux rayonnements, à la vapeur, à l'humidité, aux particules et/ou à la lumière et/ou comprend plusieurs micro-perforations (6) perméables à la vapeur ou aux gaz et/ou en ce que l'épaisseur ($d_2$) de la paroi d'enveloppe (2) augmente, de préférence de manière continue, de la paroi frontale (1) vers l'extrémité de la paroi d'enveloppe (2) éloignée de la paroi frontale (1), plus particulièrement au moins sur une zone partielle de la paroi d'enveloppe (2) et/ou

**en ce que** la paroi frontale (1) est conçue de manière circulaire et présente plus particulièrement un diamètre de 1 mm à 50 mm ou **en ce que** la paroi frontale (1) présente une forme carrée ou rectangulaire et les bords latéraux de la paroi frontale (1) présentant une longueur de 2 mm à 40 mm.

3. Capuchon de protection (10) selon la revendication 1 ou 2, **caractérisé en ce qu'**une paroi de base (3), qui prolonge la paroi d'enveloppe (2) au niveau de l'extrémité opposée à la paroi frontale (1), plus particulièrement d'une seule pièce et qui s'étend de préférence parallèlement ou avec un angle de 10° maximum par rapport à la paroi frontale (1), de la paroi d'enveloppe (2) vers l'extérieur, plus particulièrement avec une distance normale croissante par rapport à la paroi frontale (1), la paroi de base (3) présentant de préférence une forme annulaire et présentant plus particulièrement un rayon externe entre 2 mm et 50 mm et/ou un rayon interne entre 1 mm et 45 mm et/ou présente une largeur annulaire de 0,5 mm à 35 mm.

4. Capuchon de protection (10) selon l'une des revendications précédentes, **caractérisé en ce que** le capuchon de protection (10) est constitué d'un seul matériau et/ou est formé d'une seule pièce et, plus particulièrement d'une matière plastique thermoplastique ou déformée ou déformable de manière plastique, de préférence d'un des matériaux suivants ou d'un mélange de ceux-ci :

   - PETG (polyéthylènetéréphthalate glycol),
   - PP (polypropylène),
   - PE (poléthylène),
   - PC (polycarbonate),
   - PVC (polychlorure de vinyle),
   - PS (polystyrène),
   - ABS (acrylonitrile butadiène styrène),
   - HDPE (polyéthylène haute densité),
   - LDPE (polyéthylène basse densité),
   - PET (polyéthylène téréphtahalate),
   - PMMA (polyméthyl méthacrylate),
   - ECOTERM S 900 T1,
   - PETG (copolyester 67639,

le matériau du capuchon de protection (10) contient, plus particulièrement en plus, un ou plusieurs composants suivants :

   - additifs,
   - stabilisateurs,
   - colorants,
   - charges,
   - matériaux de renfort,

plus particulièrement le matériau du capuchon de protection (10) comprenant un colorant et la paroi frontale (1) étant conçue de façon à être opaque ou à absorber certaines parts de longueurs d'onde, plus particulièrement visibles.

5. Capuchon de protection (10) selon l'une des revendications précédentes, **caractérisé en ce que** l'épaisseur de la paroi d'enveloppe (2) présente, plus particulièrement, dans la direction axiale ou radiale, une extension continue, sans plis ni arêtes, sur toute la paroi d'enveloppe (2) et/ou

l'épaisseur ($d_1$, $d_2$, $d_3$) est égale à l'épaisseur moyenne dans la portion (7, 8, 9) correspondante de la paroi d'enveloppe (2) et/ou

**en ce que** la paroi d'enveloppe (2), plus particulièrement à l'exception de la zone des saillies d'encliquetage (5a) et/ou des évidements d'encliquetage (5b), est conçue avec une symétrie de rotation, et/ou

**en ce que** les épaisseurs ($d_1$, $d_2$, $d_3$) de la première portion d'épaississement (7), de la portion intermédiaire (8) et de la deuxième portion d'épaississement (9) présentent entre elles les rapports suivants :

$$2 < d_1{:}\, d_2 < 5 \text{ et/ou } 2 < d_3{:}d_2 < 5 \text{ et/ou } 0,8 < d_1{:}d_3 < 1,25$$

et/ou **en ce que** les épaisseurs ($d_1$, $d_2$, $d_3$) de la première portion d'épaississement (7), de la portion intermédiaire (8) et de la deuxième portion d'épaississement (9) sont déterminées comme suit :

$$150\ \mu m < d_1 < 250\ \mu m \text{ et/ou } 50\ \mu m < d_2 < 100\ \mu m \text{ et/ou } 150\ \mu m < d_3 < 250\ \mu m$$ et/ou en ce que la hauteur ($h_1$) de la première portion d'épaississement (7), la hauteur ($h_2$) de la portion intermédiaire (8) ainsi que la hauteur ($h_3$) de la deuxième portion d'épaississement (9) présentent entre elles les rapports suivants :

$$0,2 < h_1{:}h_2 < 0,6 \text{ et/ou } 0,8 < h_3{:}h_2 < 1,25 \text{ et/ou } 0,2 < h_1{:}h_3 < 0,6 \text{ et/ou}$$

- **en ce que** la paroi d'enveloppe présente une hauteur de 2 mm à 80 mm et/ou
- **en ce que** le rapport entre la hauteur et la dimension maximale, plus particulièrement le diamètre ou la diagonale, de la paroi frontale (1) est entre 0,01 et 55.

6. Appareil de mesure (20), plus particulièrement pour une analyse de la peau, qui peut être recouvert d'un capuchon de protection (10), le capuchon de protection (10) comprenant des évidements d'encliquetage (5a) et/ou des saillies d'encliquetage (5b), le capuchon de protection (10) étant conçu de préférence selon l'une des revendications 1 à 3, l'appareil de mesure (20) comprenant un boîtier (26) et un capteur (23) disposé à l'intérieur, le boîtier (26) comprenant une partie de boîtier (21) de forme conique avec une face frontale avant (22), au niveau de laquelle se trouve une pluralité d'évidements d'encliquetage (23a) et/ou de saillies d'encliquetage (25b) pour un encliquetage avec les évidements d'encliquetage (5a) et/ou les saillies d'encliquetage (5b) du capuchon de protection (10), plus particulièrement de façon à ce que, dans l'état encliqueté, la paroi frontale (1) du capuchon de protection (10) s'appuie contre la face frontale avant (22),

- l'appareil de mesure (20) comprenant un élément d'éjection qui est disposé dans l'extrémité de la partie de boîtier (21) de forme conique, éloignée de la paroi frontale avant (22) et qui est plus éloignée de la paroi frontale avant (22) que les évidements d'encliquetage (25a) et/ou les saillies d'encliquetage (25b),
- l'élément d'éjection (24) étant conçu pour pousser un capuchon de protection (10) encliqueté en comprimant le capuchon de protection (10) en direction de la paroi frontale avant (22) et/ou
- l'élément d'éjection étant logé de manière coulissante sur le boîtier en direction de la paroi frontale avant (22),
- une extension à levier (27) prolongeant l'élément d'éjection (24), ou une extension à levier s'écartant de l'élément d'éjection ou l'élément d'éjection (24) étant relié à une extension à levier (27),
- l'extension à levier (27) étant reliée, au centre, de manière pivotante avec le boîtier (26) de façon à obtenir un levier à deux bras à une extrémité duquel se trouve l'élément d'éjection (24),
- l'extension à levier (27) articulée au niveau du boîtier comprimant, lorsqu'il est pivoté, l'élément d'éjection (24) en direction de la paroi frontale avant (22), **caractérisé en ce que** l'élément d'éjection (24) est conçu de manière annulaire, plus particulièrement de manière cylindrique annulaire et entoure la partie de boîtier (21) de forme conique.

7. Appareil de mesure (20) selon la revendication 6, **caractérisé en ce que** l'extension à levier (27) articulée au niveau du boîtier comprime, lors de son pivotement, par l'application d'une force sur l'élément d'actionnement (28), l'élément d'éjection (24) en direction de la paroi frontale avant (22).

**8.** Appareil de mesure (20) selon l'une des revendications 6 à 7, **caractérisé en ce que** l'élément d'éjection (24) est disposé au niveau d'une extrémité du levier à deux bras et un élément d'actionnement (28) est prévu à l'autre extrémité du levier à deux bras,
plus particulièrement l'élément d'éjection (24) et/ou l'extension de levier (27) étant actionné par un élément à ressort (29) avec une force ou une précontrainte, qui comprime ou tire l'élément d'éjection (24) loin de la paroi frontale avant (22).

**9.** Appareil de mesure (20) selon l'une des revendications 6 à 8, **caractérisé en ce que** les différents évidements d'encliquetage (25a) et/ou les différentes saillies d'encliquetage (25b) présentent chacun la même distance normale par rapport à la face frontale (22) et/ou sont répartis uniformément dans la direction périphérique de la paroi d'enveloppe (2) et/ou le nombre total d'évidements d'encliquetage (25a) et/ou de saillies d'encliquetage (25b) est de deux ou quatre, et/ou
**en ce que** les évidements d'encliquetage (25a) et/ou les saillies d'encliquetage (25b) s'éloignent, avec un angle de 10° à 30° et/ou de 1 mm maximum, plus particulièrement de 0,5 mm maximum, de la paroi d'enveloppe (2) et/ou
**en ce que** deux évidements d'encliquetage (25a) et/ou saillies d'encliquetage (25b) sont prévus, qui présentent la même distance par rapport à un point dans la zone dans laquelle l'extension à levier (27) se raccorde à l'élément d'éjection (24) ou s'éloigne de celui-ci.

**10.** Procédé de fabrication d'un capuchon de protection (10), plus particulièrement selon l'une des revendications 1 à 5,

a) un film de matière plastique (31), plus particulièrement un film constitué de PETG (polyéthylènetéréphthalate glycol), étant utilisé comme matériau de départ pour un processus d'extrusion, qui présente de préférence une épaisseur entre 0,04 mm et 0,5 mm,
b) un poinçon (32), avec plusieurs poinçons partiels (33) mobiles séparément, appuyés les uns contre les autres, étant comprimé contre le film de matière plastique (31), les différents poinçons partiels (33) étant déplacés de manière normale par rapport au plan du film de matière plastique (31) et le film de matière plastique (31) étant comprimé par la pression des poinçons partiels (33) dans une contre-forme et est déformé afin d'obtenir une forme de capuchon, **caractérisé en ce que**
c) les différents poinçons partiels (33) du poinçon (32) sont écartés les uns des autres pendant ou après l'étape b), chacun des poinçons partiels (33) étant déplacé radialement vers l'extérieur à partir de la position du poinçon (32), ce qui permet de réaliser, dans le film de matière plastique (31), une paroi frontale (1), plus particulièrement avec une épaisseur de l'ordre de moins de 100 $\mu$m, de préférence entre 3 $\mu$m et 20 $\mu$m, plus particulièrement entre 5 $\mu$m et 10 $\mu$m.

**11.** Procédé selon la revendication 10, **caractérisé en ce que**, après l'étape c), les poinçons partiels (33) sont retirés de la forme de capuchon réalisée et un poinçon supplémentaire (36), avec une face frontale plate (35) et un corps de poinçon qui s'élargit, est inséré dans un volume (39) du film de matière plastique (31) formé par les poinçons partiels (33), le film de matière plastique (31) étant amené entre le poinçon supplémentaire (36) et une deuxième contre-forme afin d'obtenir une forme de capuchon,
de préférence le film de matière plastique (31), le poinçon supplémentaire (36) ou la deuxième contre-forme est poussé en avant, après le chauffage du film de matière plastique (31) à une température entre 50°C et 90°C.

**12.** Procédé selon la revendication 10 ou 11, **caractérisé en ce que**, pour le réglage de l'épaisseur ($d_1$, $d_2$, $d_3$) de la paroi d'enveloppe (2) s'éloignant de la paroi frontale (1), la vitesse d'avance (v) et/ou la température des poinçons partiels (33), du poinçon supplémentaire (36) et/ou la température du film de matière plastique (31) et/ou la température de la première ou de la deuxième contre-forme est réglée,
pour la réduction de l'épaisseur de paroi au niveau du bord (36) de l'évidement formé par le poinçon supplémentaire, une augmentation de la température ainsi qu'une augmentation de la vitesse d'avance étant prévues, et
pour l'augmentation de l'épaisseur de paroi au niveau du bord (36) de l'évidement formé par le poinçon supplémentaire, une réduction de la température mentionnée ainsi qu'une réduction de la vitesse d'avance étant prévues.

**13.** Procédé selon l'une des revendications 10 à 12, **caractérisé en ce que** la température du poinçon supplémentaire (36) et/ou du film de matière plastique (31) ainsi que la vitesse d'avance du poinçon supplémentaire (36) est réglé de façon à ce que
pendant une première période d'avance du poinçon supplémentaire (36), une portion de la paroi d'enveloppe (2) soit réalisée avec une épaisseur ($d_2$) prédéterminée, plus particulièrement de 50 $\mu$m à 100 $\mu$m, et
pendant une deuxième période d'avance suivant la première période d'avance du poinçon supplémentaire (36), une portion de la paroi d'enveloppe (2) soit réalisée avec une épaisseur prédéterminée ($d_3$), plus particulièrement

de 150 $\mu$m et 250 $\mu$m, qui est supérieure à l'épaisseur ($d_2$) de la paroi d'enveloppe (2) créée lors de la première période d'avance,

plus particulièrement l'épaisseur de paroi ($d_2$) soit réglée, pendant la première période d'avance du poinçon supplémentaire (36), à une valeur qui correspond du double au quintuple de l'épaisseur de paroi ($d_1$) de la zone d'enveloppe qui a été formée lors de la déformation de la paroi frontale (1) par l'application des poinçons partiels (33).

14. Procédé selon l'une des revendications 10 à 13, **caractérisé en ce que** le film de matière plastique (31) est constitué d'une matière thermoplastique ou déformé ou pouvant être déformée de manière plastique, de préférence d'un des matériaux suivants ou d'un mélange de ceux-ci :

   - PETG (polyéthylènetéréphthalate glycol),
   - PP (polypropylène),
   - PE (poléthylène),
   - PC (polycarbonate),
   - PVC (polychlorure de vinyle),
   - PS (polystyrène),
   - ABS (acrylonitrile butadiène styrène),
   - HDPE (polyéthylène haute densité),
   - LDPE (polyéthylène basse densité),
   - PET (polyéthylène téréphtahalate),
   - PMMA (polyméthyl méthacrylate),
   - ECOTERM S 900 T1,
   - PETG (copolyester 67639,

le matériau du capuchon de protection (10) contient, plus particulièrement en plus, un ou plusieurs composants suivants :

   - additifs,
   - stabilisateurs,
   - colorants,
   - charges,
   - matériaux de renfort,

plus particulièrement le matériau du capuchon de protection (10) comprenant un colorant et la paroi frontale (1) étant conçue de façon à être opaque ou à absorber certaines parts de longueurs d'onde, plus particulièrement visibles.

15. Procédé selon l'une des revendications 10 à 14, **caractérisé en ce que**

   - les poinçons partiels (33) sont déplacés en rotation, pendant leur mouvement radialement loin de son centre, à l'étape c), le long d'une courbe s'étendant en spirale vers l'extérieur autour d'un axe central (X) et s'élargissant, et/ou
   - après la déformation partielle ou entière de la paroi d'enveloppe (2), les poinçons partiels (33) ou le poinçon supplémentaire (36) sont reculés et le volume (39) déformé est alimenté, plus particulièrement avec de l'air comprimé chauffé et **en ce que** le poinçon supplémentaire (36) est ensuite inséré, le cas échéant, dans le volume déformé (39) pour la réalisation de l'étape de déformation suivante, plus particulièrement, grâce à l'application d'air comprimé, des évidements d'encliquetage (5b) étant déformés dans l'enveloppe interne de la paroi d'enveloppe (2) et/ou **en ce que**, après la déformation de la paroi d'enveloppe (2), le film de matière plastique (31) restant prolongeant la paroi d'enveloppe (2) est séparé de la paroi d'enveloppe (2) le long d'une ligne de découpe (37), la ligne de découpe (37) étant choisie plus particulièrement de façon à ce qu'une portion du film de matière plastique (31) restant prolongeant la paroi d'enveloppe (2) se trouve à l'intérieur de la ligne de découpe et forme une paroi de base (3) et/ou
   - **en ce que** le film de matière plastique (31), plus particulièrement après sa déformation, est chauffée, après sa déformation, pendant une période prédéterminée, plus particulièrement pendant au moins 3 secondes, à une température prédéterminée entre 30°C et 120°C, plus particulièrement à une température entre 50°c et 90°C, et/ou

**en ce que** les poinçons partiels (33) et/ou le poinçon supplémentaire (36) et/ou les contre-formes utilisées sont chauffées, avant le traitement du film de matière plastique (31), à une température entre 30°C et 120°C, plus particulièrement à une température entre 50°C et 90°C et, pendant le traitement, maintenu à une température comparable et/ou **en ce que**, plus particulièrement, au moyen d'un laser ou d'une micro-aiguille ou d'une nano-aiguille chauffée ou par gravure, des trous (6), plus particulièrement des micro-perforations ou des nano-perforations (6), sont réalisés dans la paroi frontale (1).

Fig. 1b

Fig. 1a

Fig. 2

Fig. 3

Fig. 4

Fig. 4a

EP 2 858 568 B1

Fig. 5

Fig. 5a

Fig. 6

EP 2 858 568 B1

Fig. 7b            Fig. 7            Fig. 7a

EP 2 858 568 B1

Fig. 8

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2010329951 A
- EP 1262753 A